# EUROPEAN PATENT APPLICATION

(11) **EP 2 481 413 A1**
(43) Date of publication of application: **01.08.2012**
(21) Application number: 10819101.6
(22) Date of filing: 21.09.2010
(51) Int. Cl.: A61K 31/7016, A61K 31/702, A61K 36/00, A61P 1/00

(54) **VETERINARY PHARMACEUTICAL COMPOSITION AND METHOD (ALTERNATIVES) FOR THE PROPHYLAXIS AND TREATMENT OF DISEASES OF THE GASTROINTESTINAL TRACT AND INTOXICATIONS OF DIVERSE ETIOLOGY IN ANIMALS**

(30) Priority: 23.09.2009 RU 2009135348
(71) Applicant: Dikovskiy, Aleksander Vladimirovich, Moscow 123182 (RU)
(72) Inventor: TRETYAKOV, S rg i Vikt r vi h, Li tskaya obl. 399140 (RU); TURCHEV, l g l ks ndr vi h, s w 121609 (RU)
(74) Representative: Ackermann, Joachim
(86) International application number: PCT/RU2010/000520
(87) International publication number: WO 2011/037495

(57) **Abstract**

A veternary pharmaceutical composition for treating diseases of the gastrointestinal tract and intoxications of diverse etiology in animals comprises, as the active component, hydrolyzed lignin and a prebiotic from the following group: lactulose, fructooligosaccharides, galacto-saccharides and inulin, and is in a form suitable for use in the composition of a mixture with combined feed, with a lignin content of from 30 to 95 % by mass, in the form of powder of granules. The method for the prophylaxis and treatment of intoxication of diverse etiology, including mycotoxicoses, in animals, including birds, envisages that the animal will receive the above-mentioned veterinary pharmaceutical composition mixed with feed in a dosage of from 2 to 4.5 kg of the preparation per ton of combined feed, proportional to the content of mycotoxins in the combined feed. The method for the prophylaxis and treatment of diseases of the gastrointestinal tract, including dyspepsia, gastroenteritis, enteritis, colitis, hepatitis, and hepato-dystrophy, including in a toxic form, in animals, including cattle and pigs, envisages that the animal will receive the veterinary pharmaceutical composition according to claim 1 30 ± 10 minutes prior to feeding, perorally individually or in a group method with water for watering or with the feed, 1 to 2 times per day in a dosage of from 0.2 to 0.3 g/kg of the mass of the animal. This ensures an increase in the effectiveness of the treatment and elimination of the above-mentioned defects in the use of hydrolyzed lignin, activated carbon and other generally known sorbents. At the same time, the joint use of lignin with a prebiotic as a means for the prophylaxis and treatment of animals makes it possible to produce a result in the form of a set of positive effects: minimization of side effects from the use of a sorbent; restoration of homeostasis in the gastrointestinal tract; and normalization of the metabolism indices, this finally resulting in a qualitative increase in the zootechnical and economic indices of animal husbandry.

## Description

The claimed group of inventions is related to medicine, specifically, veterinary medicine, and can be used for prophylaxis and treatment of gastrointestinal tract (GIT) diseases and intoxications of different etiologies, inclusive of mycotoxicosis, dyspepsia, gastroenteritis, enteritis, colitis, hepatitis or hepatic dystrophy (including its toxic form) in animals including poultry, cattle and pigs.

In conditions of intensive animal husbandry and expansion of intensive animal farming, along with the improving of animal breeding and productivity, a particular attention must be paid to the increase of survival of young animals, as well as to increase in animal productivity. Different animal diseases, primarily gastrointestinal disorders in the form of acute and chronic digestive disorders, lead to the increase in mortality of young livestock, such as calves and pigs. Widespread livestock diseases bring the enormous economic losses resulting from young livestock mortality and pronounced reduction of productivity both during animal growth stage and after the convalescent animals have reached full maturity.

This problem is very difficult to solve because of many causative agents involved in animal pathology and its widespread occurrence. In such circumstances a search for already available and potent therapeutic and prophylactic compounds against animal diseases is required.

Data published in the literature on the basis of studies within and outside of Belarus has shown that the unfavorable environmental conditions, noncompliance with technology of animal feeding, housing and use and uncontrolled administration of medicinal products lead to homeostasis disorders triggering destructive body changes.

Non-infectious diseases play a significant role in a variety of animal pathological conditions. Diseases of young livestock have a special place in animal pathology. When the young livestock succumbs to disease in the early postnatal period, this is the evidence that the breeding stock is likely to have some metabolic diseases. Besides, it should be taken into account that the metabolic rate of growing animals is high, and, hence, they are very sensitive to violations of housing and feeding requirements.

The normal functioning of live organisms takes place only when the condition of the internal milieu is optimal (Mityushin V.V. Dyspepsia in newborn calves. - M.: Rosagropromizdat, 1989. - 123 pp.; Karput I.M., Pivovar L.M., Ulyanov A.G., Scheglov V.M. Pathomorphology and diagnosis of autoimmune disorders of gastrointestinal system in animals. // Problems of pathomorphological diagnosis of diseases in animal husbandry. Materials of the All-Union scientific conference, Vilnius, 1986, p. 172-173).

A concept of endogenous intoxication has been coined fairly recently in veterinary literature.

Endotoxicosis is an intricate pathogenetic complex which includes the metabolic and functional disorders in virtually all organs and body systems, and is observed in many diseases. The mechanism of endotoxicosis is based on the preponderance of catabolic over anabolic processes which lead to decompensation of body regulatory systems and accumulation in toxic concentrations of their effector components, such as proteolytic enzymes, kinins and other vasoactive peptides, biologically active products of protein dehydration, inflammatory mediators, etc.

From the literature data (Abramov S.S. et al. General animal therapy. - Vitebsk: UO VGAVM, 2006 - 217 pp.; Karput I.M., Pivovar L.M., Ulyanov A.G., Scheglov V.M. Pathomorphology and diagnosis of autoimmune disorders of gastrointestinal system in animals. // Problems of pathomorphological diagnosis of diseases in animal husbandry. Materials of the All-Union scientific conference, Vilnius, 1986, p. 172-173) it is known that the newborn and growing animals are most susceptible to the advancing intoxication. One of the reasons of such high susceptibility of newborn animals is that, unlike humans, during the first days of animal life a fraction of blood is flowing from the gut bypassing the hepatic capillary network through the open venous duct and falls into the posterior vena cava, and subsequently into the circulation. Excessive accumulation of toxins in the body of young livestock, as well as the inability of physiological detoxification systems to secure the effective elimination of toxins, predetermine the necessity of intensive detoxification therapy using specific agents and methods of detoxification.

Another important problem of the animal husbandry industry worldwide is the toxicoses of various etiologies, among which the mycotoxicoses constitute a widespread problem. According to the annual literature survey, at least 25 - 30% of the compound animal feedstuff is contaminated with mycotoxins. The situation is seriously compounded by the fact that the modem livestock breeds and poultry crosses are highly susceptible to negative environmental factors, which collectively brings significant economic losses should a disease occur and take a chronic course. Besides, many toxins, including the mycotoxins, can be passed on through food originally from an animal source, possess mutagenic and carcinogenic properties or affect human psychophysiology and behavior.

The methods used for body detoxification have been known even to our ancestors who used ash, charcoal or some clayish alumosilicates to neutralize toxic effects. Of those, only charcoal had stood the test of time, and got passed to the 70^{th}-80^{th} of the last century as an officially registered and certified activated charcoal. Now it is a well known and widely used in medical practice medication. Further works on the synthesis and investigation of microspherical enterosorbents created on the basis of synthetic activated charcoals prompted their refinement and implementation into medical practice. Use of powdered activated carbon sorbents enabled to significantly reduce the damaging effect of charcoals on gastrointestinal mucous membrane. By the end of the 80^{th} - mid-90^{th}, apart from the modified charcoal compounds, the enterosorbents based on highly dispersed silicon oxide (Polysorb), pyrolized cellulose filaments (Polyphepan) and some other natural and synthetic polymers had been approved for use and implemented in medical practice. There are reports on the use of lignin, fermasorb, zoosorb, plantosil and SV-1 as enterosorbents in veterinary medicine.

The lignin-containing pharmaceutical compositions are known to be used for treatment of animals (SU Nº 803157, RU Nº 2096033, 2141329, 2302866).

However, all known compositions do not have the required therapeutic properties, and their based treatment is not sufficiently effective.

There is a pharmaceutical composition and method of prophylaxis and treatment of gastrointestinal animal diseases by oral administration of enterosorbent, the hydrolyzed lignin in the form of water suspension (prototype - RU Nº 2297214).

However, despite this group of compounds is beneficial in terms of sorption of toxins, they have an important negative impact on animal husbandry, namely, their inclusion into the animal feed ration in quantities sufficient for effective sorption of toxins is accompanied by a simultaneous sorption of a fraction of biologically active substances from the feedstuff (vitamins, essential amino acids, mineral elements, etc.) which adversely affects various production parameters including growth of animals and poultry. The method of enterosorbent administration in the form of water suspension is hardly suitable for the broad implementation in animal husbandry practices.

The technical objective of a group of inventions bound together by a common concept is to create an effective pharmaceutical composition for prophylaxis and treatment of gastrointestinal diseases and intoxications of different etiologies in animals, as well as the methods of treatment using the abovementioned pharmaceutical composition.

The technical result which enables to solve the assigned task is the increased efficacy of treatment and correction of the above disadvantages of using hydrolyzed lignin, activated charcoal and other well-known sorbents. At the same time, a co-administration of lignin with a prebiotic as a means for prophylaxis and treatment of animals enable to achieve a complex result with the following positive effects: minimization of sorbent-related side effects, recovery of homeostasis of gastrointestinal tract, normalization of metabolic parameters, and eventually the increase of the livestock productivity and economic performance values.

The essence of invention regarding the veterinary pharmaceutical composition for treating diseases of the gastrointestinal tract and intoxications of various etiologies in animals, is that it comprises, as the active components, hydrolyzed lignin and a prebiotic from the following group: lactulose, fructooligosaccharides, galactosaccharides and inulin, and is in a form suitable for use in the complex feed mixture with compound feed, with lignin content from 30 to 95% by mass and a prebiotic content from 5 to 50% by mass, preferably in the form of powder or granules.

The essence of invention regarding the prophylaxis and treatment of intoxications of various etiologies, including mycotoxicoses in animals, inclusive of birds, is that the animal will receive the abovementioned veterinary pharmaceutical composition according to claim 1, mixed with the compound feedstuff at a dose from 2 to 4.5 kg of the preparation per 1 ton of compound feedstuff, proportionally to the content of mycotoxins in the feed.

The essence of invention regarding the method for the prophylaxis and treatment of diseases of the gastrointestinal tract, inclusive of dyspepsia, gastroenteritis, enteritis, colitis, hepatitis and hepatic dystrophy, including that in a toxic form, in animals, including cattle and pigs, is that the animal will receive the veterinary pharmaceutical composition according to claim 1, 30 ± 10 minutes prior to feeding, orally, either individually or using a group method, with water for watering or with the feedstuff, 1 - 2 times a day at a dose from 0.2 to 0.3 g/kg of animal body weight.

The claimed pharmaceutical composition is intended for implementation under the trade name "Ecofiltrum" and can be used as a water suspension, or by mixing the combination of lignin/prebiotic with the feedstuff which is more convenient for use.

The examples of implementation of invention are given below.

Composition 1 "Ecofiltrum 800 + 200":
- Lignin hydrolyzed - 80%;
- Lactulose - 2.0%.

Composition 2 "Ecofiltrum 900 + 100":
- Lignin hydrolyzed - 90%;
- Lactulose - 10%.

The below given experimental results illustrating the use of the composition and method(s) confirm the possibility of achieving the indicated technical result.
Figure 1 demonstrates the macroscopic picture of piglet liver with hepatic dystrophy,
Fig. 2 shows a healthy piglet liver (A) and liver affected with hepatic dystrophy (B),
Fig. 3 shows the microscopic picture of piglet liver affected with hepatitis,
Fig. 4 shows the histological section of piglet liver affected with hepatic dystrophy (granular, fatty and hydropic dystrophy, marked discomplexing of hepatic tubules, necrobiosis and necrosis ofhepatocytes, hematoxylin-eosin staining (10 x 20),
Fig. 5 shows histological section of the Ecofiltrum - treated piglet liver affected with hepatic dystrophy (fatty small-globule dystrophy, insignificant focal lymphoid macrophagal interstitial infiltrates, discomplexing of hepatic tubules, hematoxylin-eosin staining (10 x 15),
Fig. 6 shows histological section of liver of the piglet affected with gastroenteritis and hepatic dystrophy (granular and fatty (small-globule) dystrophy of hepatocytes, dilatation of the inter-tubular sinusoids, hematoxylin-eosin staining (10 x 30),
Fig. 7 shows histological section of liver of the Ecofiltrum - treated piglet affected with gastroenteritis, hematoxylin-eosin staining (10 x 20). There are insignificant lymphocytic-macrophagal infiltrates among the hepatic tubules, insignificant dilatation of hepatic spaces, granular dystrophy of hepatocytes, hematoxylin-eosin staining (10 x 30),
Fig. 8 shows histological section of liver of the piglet affected with hepatitis (large focal lymphocytic-macrophagal proliferates, necrobiosis and necrosis of hepatocytes, granular dystrophy of hepatocytes, discomplexing of hepatic tubules, hematoxylin-eosin staining (10 x 25),
Fig. 9 shows histological section of liver of the Ecofiltrum - treated piglet affected with hepatitis. There are insignificant lymphocytic-macrophagal proliferates. discomplexing of hepatic tubules at places and granular dystrophy of hepatocytes. Hematoxylin-eosin staining (10 x 25).

### 1. Experiment 1.

Composition of "Ecofiltrum" has been studied. The study objects were 1-month old piglets. The study subjects were clinical condition, clinical trial parameters, basic metabolic panel parameters and results of anatomo-pathological and histological examinations.

The main study objective was to evaluate the therapeutic efficacy of Ecofiltrum for gastroenteritis, toxic hepatic dystrophy and hepatitis in pigs.

A complex of clinical, hematological, biochemical, anatomo-pathological and histological methods was used in the study.

The scientific novelty of the study was that for the first time the therapeutic efficacy of Ecofiltrum was studied for gastroenteritis, toxic hepatic dystrophy and hepatitis in piglets.

### 1.1. Materials and methods

Studies were carried out in December 2007 and January-March 2008 at the pig breeding complex RSUP "Jubilee Agrokombinat" of Orsha district of Vitebsk region, as well as the district veterinary laboratory, laboratory of the Department of anatomo-pathology and histology and diagnostic department of scientific research institute of "Applied veterinary medicine and biotechnology" of UO VGAVM.

One-month old sick piglets were divided into 4 groups of 15 animals each. Group 1 comprised pigs with toxic hepatic dystrophy, group 2 - pigs with hepatitis and group 3 - pigs with gastroenteritis. Groups were formed based on the conditional analogue principle. Groups were assembled gradually as pig morbidity was increasing. Group 4 comprised healthy pigs of the same age.

Clinical manifestations of hepatic dystrophy in piglets included general depression, short periods of light-brown thin stool, muscular weakness, periodic convulsions, vomiting, anorexia and sometimes acrocyanosis. Growth and development of animals in poor health were retarded as compared to healthy pigs of the same age. Piglets with hepatitis demonstrated diarrhea, depression (down to the semi-comatose condition), loss of appetite (down to anorexia), tachycardia and dyspnea. Their body weight was reduced although not so significantly as in pigs with hepatic dystrophy. Feces color changed to pale or clay-colored.

Gastroenteritis in piglets was clinically manifested in depression, loss of appetite and thirst. Sick animals gathered in small groups, with their hair ruffled, and a fraction of animals was lying. Their particular behavioral reactions were clearly traceable during feeding when sick animals actively ate the first portions, then quickly walked away from the feeder and stood for some time with their heads down and legs straddled, indicating that they experienced symptoms of gastralgia. They had increased stool frequency, with feces from pale-yellow to dark-grey color, containing mucous and sometimes blood streaks, and having sourish-putrid smell.

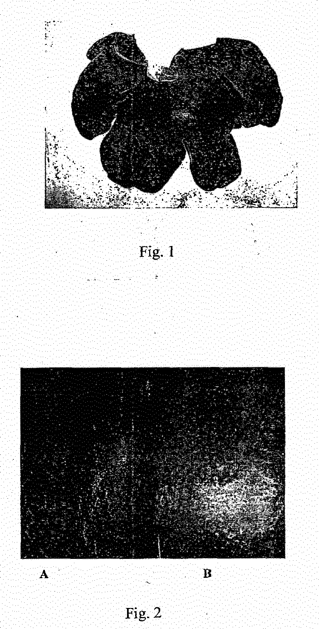

The above indicated diseases could be differentiated more accurately upon autopsy and anatomical examination of the involuntarily slaughtered pigs with typical clinical signs of hepatic dystrophy, hepatitis or gastroenteritis, or after histological examination of the liver [A.V. Zharov, 1984; A.V. Senko et al., 2001]. Thus, in case of hepatic dystrophy pig's liver was slightly enlarged, of flabby consistence and bright or ochrous yellow color (Fig. 1).

One of the most typical signs of hepatic dystrophy is liver mottling (mosaic appearance). The liver has a light brown or yellowish-cherry red color. The sites of different color are unevenly scattered. Such changes are more pronounced on the diaphragmatic surface of the liver. The dark red sites have a well discernible lobular structure, and there is a pronounced vascularity of the central part of the lobules owing to the dilatation of central veins at that (Fig. 2).

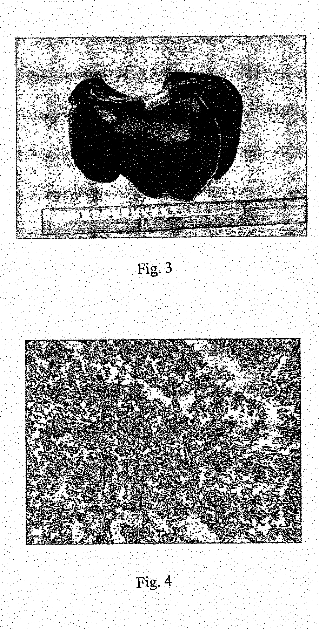

In parenchymatous hepatitis the vascularity of the liver is well pronounced. Portal lymph nodes are reactive. The flabbiness and roughness of the liver are less pronounced, and its color is either grey-clayish or dark red (Fig. 3).

Gastroenteritis is also accompanied by dystrophic processes in the liver. At the same time, there are dystrophic changes in the myocardium and kidneys. Gastric mucosa looks swollen, loose, hyperemic, sometimes with hemorrhages, and coated with viscous mucous. Erosions and ulcerations can be observed.

Pigs of all groups were bled for hematological and biochemical blood analysis before the beginning of treatment and after their recovery. Blood was examined for basic metabolic panel parameters which can be conditionally divided into the following groups of tests (Table 1):
- Tests for metabolism of pigments, proteins, carbohydrates, lipids, vitamins and minerals;
- Tests for control of activity of specific liver enzymes;
- Tests for detoxifying function;
- Tests for analysis of excretory function.

**Table 1. Blood test parameters and methods used in experiment 1**

| *Parameters* | Methods |
|---|---|
| 1. Erythrocytes | Automated hematology analyzer Medonic 620 CA (Sweden). |
| 2. Leukocytes | |
| 3. Hemoglobin | |
| 4. Hematocrit | |
| 5. MCV | |
| 6. MCH | |
| 7. MCHC | |
| 8.PLT | |
| 9. MPV | |
| 10. Alanine aminotransferase | Automatic biochemical analyzer Cormay-Lumen (Spain). |
| 11. Aspartate aminotransferase | |
| 12. Total cholesterol | |
| 13. Total bilirubin | |
| 14. Alkaline phosphatase | |
| 15. Total protein | |
| 16. Albumins | |
| 17-19. α-, β-, γ-globulins | Electrophoretic technique using Sejbia apparatus (France). |

All sick animals were treated with "Ecofiltrum" containing hydrolyzed lignin and lactulose, by oral administration at a dose of 0.3 g per kg body weight once daily until animal recovery.

Full animal recovery was judged by the disappearance of all clinical signs of the disease, return of appetite and positive dynamics of laboratory parameters. After completion of the treatment course and clinical recovery of animals, one pig was collected from each group for control slaughter and histological examination of the autopsied material.

All laboratory tests were carried out at the diagnostic department of scientific research institute of "Applied veterinary medicine and biotechnology" and department of anatomo-pathology and histology of UO VGAVM.

### 1.2. Study results

In animals with gastroenteritis which were treated as described above, the appetite returned in 1-2 days, symptoms of dehydration (eyeball retraction, decreased skin plasticity) disappeared in 2-3 days, and diarrhea had gone in 3-4 days.

In pigs with hepatic dystrophy the disease lasted for 5 days. In animals of group 2 their treatment resulted in disappearance of clinical signs of hepatitis in 4-5 days.

Clinical blood analysis has shown that by the end of treatment period pigs of all study groups demonstrated reduction of hemoglobin concentration and low leukocyte count. Thus, the concentration of hemoglobin by the end of the experiment decreased from 94.75 ± 2.076 g/L to 89.1 ± 2.945 g/L; from 91.46 ± 3.039 g/L to 83.1 ± 1.45 g/L; from 101.5 ± 5.35 g/L to 85.6 ± 2.57 g/L (P < 0.01), and leukocyte count dropped from 26.16 ± 0.584 x 10⁹/L to 15.15 ± 0.465 x 10⁹/L; from 25.86 ± 0.548 x 10⁹/L to 16.59 ± 0.281 x 10⁹/L; from 17.9 ± 1.70 x 10⁹/L to 16.90 ± 0.229 x 10⁹/L (P < 0.01) for animal groups 1 - 3, respectively. These results attest to the recovery of blood parameters and attenuation of inflammatory events in treated animals.

More significant changes were found in the basic metabolic panel parameters. Table 2 presents the dynamics of major tests which enable to draw the conclusion about protein, pigment and lipid metabolism, as well as the activity of hepatospecific enzymes, in sick pigs which had undergone treatment.

The analysis of Table 2 allows the conclusion that treatment results in the recovery of functional ability of hepatic parenchyma. This is evidenced by decreased cholesterol level as an indication of restoration of the hitherto disturbed fat metabolism. In the course of treatment serum enzyme activity also rapidly returned to normal, that was evidenced by a significant reduction of activity of hepatospecific enzymes (AsAT, AlAT, ALP) as a result of recovery processes primarily in hepatocytes. Treated animals demonstrated a significant reduction of bilirubin levels that also attests to the attenuation of cytolytic syndrome. The recovery process was also accompanied by positive changes in serum proteinogram (the increase of albumin fraction) indicating the recovery of the albumin-synthesizing liver function with simultaneous regulation of the β- and γ-globulin levels. All these changes attest to significant decline of antigen sensitization of mesenchymal stroma of the liver in treated pigs.

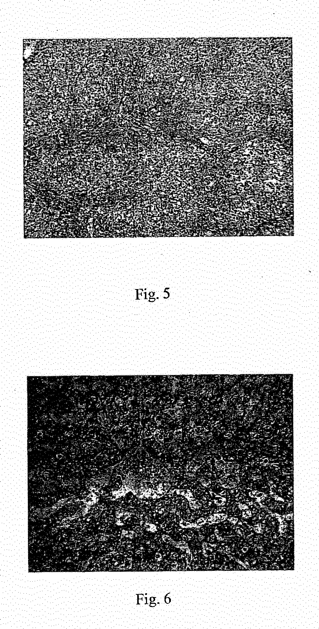

The efficacy of treatment is also confirmed by the histological examination results. Thus, if in the beginning of the disease hepatic dystrophy was manifested in severe fatty, granular and hydropic dystrophy of hepatocytes, picnosis and cariorexis, and destruction of hepatic tubules (Fig. 4) that is a typical patho-histological picture of the toxic form of the disease, [A.V. Zharov, 1984; M.S. Zharov et al., 1983; T.B. Goehring et al., 1984], then, after the administration of CB-1 enterosorbent the dystrophic processes were less pronounced (mostly small-globule, fatty dystrophy). Hepatic tubular structure was partly restored, most probably owing to the less intense infiltration of hepatic lobes with lymphocytes and macrophages (Fig. 5).

**Table 2. Dynamics of some pig serum biochemical parameters during the experiment (M ±m)**

| Parameters | Animal group number | Examination results | |
|---|---|---|---|
| | | Before treatment | After treatment |
| 1 | 2 | 3 | 4 |
| AsAT, µkat/L | 1 | 0.91 ± 0.038 | 0.47 ± 0.027* |
| | 2 | 0.81 ± 0.10 | 0.64 ± 0.009* |
| | 3 | 1.57 ± 0.065 | 0.62 ± 0.017* |
| | 4 | 0.45 ± 0.030 | |
| AlAT, µkat/L | 1 | 0.79 ± 0.046 | 0.56 ± 0.015* |
| | 2 | 1.001 ± 0.042 | 0.76 ± 0.012* |
| | 3 | 1.14 ± 0.026 | 0.71 ± 0.013* |
| | 4 | 0.57 ± 0.027 | |
| Total cholesterol, mmol/L | 1 | 2.32 ± 0.154 | 1.21 ± 0.073** |
| | 2 | 2.30 ± 0.101 | 1.09 ± 0.077** |
| | 3 | 2.03 ± 0.123 | 1.15 ± 0.043** |
| | 4 | 1.30 ± 0.090 | |
| Total bilirubin, µmol/L | 1 | 12.15 ± 0.907 | 6.72 ± 0.276** |
| | 2 | 12.89 ± 1.327 | 5.79 ± 0.247** |
| | 3 | 13.14 ± 0.740 | 5.93 ± 0.230* |
| | 4 | 5.61 ± 0.327 | |
| ALP U/L | 1 | 147.99 ± 12.332 | 81.71 ± 3.857* |
| | 2 | 133.64 ± 13.066 | 79.34 ± 2.771** |
| | 3 | 169.38 ± 16.056 | 115.1 ± 2.345* |
| | 4 | 78.87 ± 2.452 | |
| Total protein, g/L | 1 | 59.1 ± 1.01 | 56.29 ± 1.740* |
| | 2 | 49.7 ± 1.97 | 57.29 ± 2.071* |
| | 3 | 46.3 ± 1.87 | 56.56 ± 0.826* |
| | 4 | 56.7 ± 1.01 | |
| Albumins, g/L | 1 | 20.06 ± 1.02 | 24.1 ± 0.23* |
| | 2 | 18.6 ± 0.12 | 25.6 ± 0.14* |
| | 3 | 19.9 ± 0.25 | 28.6 ± 0.22** |
| | 4 | 23.3 ± 0.14 | |
| α-globulins, g/L | 1 | 12.8 ± 0.15 | 13.5 ± 0.07* |
| | 2 | 16.2 ± 0.24 | 12.1 ± 0.32* |
| | 3 | 11.1 ± 0.12 | 10.7 ± 0.21** |
| | 4 | 12.5 ± 0.08 | |
| β-globulins, g/L | 1 | 11.5 ± 0.21 | 8.8 ± 0.13** |
| | 2 | 7.2 ± 0.23 | 9.6 ± 0.08** |
| | 3 | 6.9 ± 0.14 | 6.8 ± 0.12* |
| | 4 | 7.6 ± 0.23 | |
| γ-globulins, g/L | 1 | 14.2 ± 0.23 | 10.9 ± 0.15[]* |
| | 2 | 7.7 ± 0.14 | 9.9 ± 0.21* |
| | 3 | 8.4 ± 0.09 | 9.6 ± 0.11** |
| | 4 | 9.2 ± 0.23 | |

| | | | |
|---|---|---|---|
| Note: * - P < 0.001 as compared to baseline values; ** - P < 0.01 as compared to baseline values. | | | |

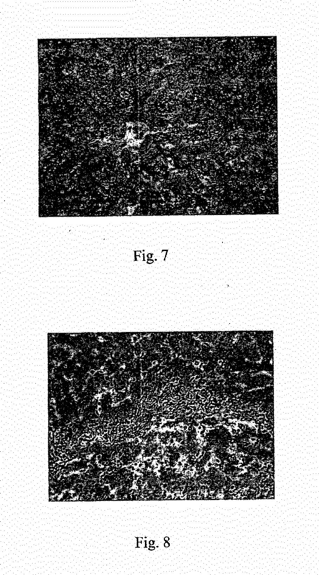

In gastroenteritis, changes in the liver are much less pronounced as compared to those in the initial stage of toxic hepatic dystrophy. Nonetheless, at magnification of 10 x 30, marked signs of granular and fatty (small-globule) dystrophy of hepatocytes were noted. Hepatic spaces (inter-trabecular sinusoids) were dilated owing to the infiltration with lymphocytes and macrophages (Fig. 6). The extent of such infiltration was significantly decreased after administration of enterosorbent as evidenced by somewhat narrowed inter-trabecular sinusoids (Fig. 7). A milder degree of granular hepatocyte dystrophy was a common sign of recovery.

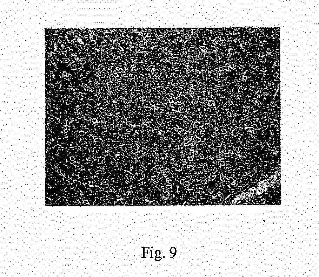

Ecofiltrum administration in piglets with hepatitis reduced the extent of inflammatory process. If at the beginning of the disease there were large focal lymphocytic-macrophagal proliferates and edema of the inter-lobular connective tissue (Fig. 8), then, after the treatment the processes of reparative regeneration of liver tissues were observed, as evidenced by scarce proliferates, fewer foci of hepatocytes' necrobiosis and discomplexing of hepatic tubules (Fig. 9).

### 1.3. Conclusions:

1. "Ecofiltrum" administered per os at a dose of 0.3 g/kg animal body weight, once daily until the disappearance of all symptoms, is a highly efficient treatment of hepatic dystrophy, hepatitis (inclusive of its toxic form) and gastroenteritis in piglets.
2. Clinical signs of hepatic dystrophy in the Ecofiltrum-treated pigs disappear, on average, in 5 days. During this period the most altered parameters in blood of sick animals are either normalized or show a steady trend towards normalization. Also, treatment leads to morphological recovery of the liver.
3. The Ecofiltrum-treated piglets recover from hepatitis, on average, in 4-5 days.
   Treatment stimulates the intensive regeneration process in the liver which is primarily due to a diminished inflammatory response. The effect of treatment is manifested in the reduction of leukocyte count and lower intensity of cytolysis in hepatocytes.
4. Symptoms of diarrhea and dehydration in piglets affected with gastroenteritis disappear in 3-4 days since the beginning of Ecofiltrum treatment. Restoration of digestion and intestinal absorption bring the normalization of protein metabolism, as well as the morphological structure and functional state of the liver.

Therefore, based on the results of therapeutic efficacy and positive dynamics of biochemical blood parameters, it can be concluded that "Ecofiltrum" is an effective in means of pathogenetic therapy for treatment of pigs suffering from toxic hepatic dystrophy, hepatitis and gastroenteritis.

### Experiment 2.

A new scientific study was carried out. The objective of this experimental study was to investigate the effectiveness of use of the complex preparation "Ecofiltrum" included in the ration of broiler chickens, aimed at prophylaxis of combined forms of chronic mycotoxicoses in comparison with the existing analogous preparations, and to determine the optimal composition and dosage of "Ecofiltrum".

### 2.1. STUDY MATERIAL AND METHODS

In accordance with study objective, this scientific and applied experiment was carried out at the animal house of the ONO "Zagorsk EPH VNITIP". The experiments were carried out using broiler chickens of the "Cobb-Avian-48" cross. A total of 7 groups have been formed (2 control groups and 5 experimental groups, 38 birds in each) based on the conditional analogue principle. The birds were fed ad libitum using the balanced dry compound feed with the parameters of feed nutrition set in accordance with the recommended standards of bird feeding by VNITIP (2006). The composition and parameters of the compound feed are given in Appendix 1 (page 39 ). Until the 5-day age all chickens received a prestarting "zero" ration, and starting from the 6-day age they were fed the experimental mixed feeds. The birds' housing conditions were in accordance with the accepted zoohygienic parameters. The duration of the experiment was 5 weeks (35 days).

In vast majority of cases mycotoxins are constantly found out in raw feed and cause an economic loss to farms even in small amounts. The analysis of literary data has shown that one of the most efficient ways of protection against mycotoxins is the use of sorbent preparations. Undoubtedly the usage of enterosorbents as an element of an afferent therapy and a self-contained method of intracorporal detoxication obviously has promising perspectives because the binding of toxic substances in the gastrointestinal tract of animals and birds and the decrease of their concentration of blood helps to efficiently improve the health status of livestock. This is the physiological basis of the enterosorbents' positive effect when animals are treated for chronic diseases.

At the same time, lately a growing importance is assigned to the normal flora as a protective agent against the exogenous toxins and endogenous substrates and metabolites. Given the importance of the gastrointestinal tract, the particular attention should be paid to the intestinal bacteria playing a role of the primary barrier or a "metabolic organ" protecting against intrusion of foreign substances from the digestive tract. The intestinal flora plays a role of a biosorbent and, along with the natural enzymatic and immune systems, takes part in detoxification of xenobiotics, i.e., creates a complex of mechanisms not only preventing the absorbance of toxic substances but also partaking in their microbiological destruction. In this context, in order to reduce consequences of chronic mycotoxicosis and achieve of the maximal protection of the organism it is expedient to complement the enterosorbent treatment with prebiotics added to the feedstuff. Prebiotics are the non-digestible food ingredients which improve the trophic status and non-specifically stimulate the population of adapted forms of symbiotic microflora.

In practical conditions it is particularly important to take into account the fact that the administration of only one medication against numerous disorders is often quite inefficient, and its feeding at maximum doses is fraught with the whole range of adverse effects. At the same time, the possibility of a better control over toxic events can be secured by using a reasonable combination of substances. Such an approach seems to be reasonable from many standpoints. Nonetheless, of principal importance is not the very fact of declared use of several biologically active substances at once and within the same preparation, but rather the determination of their effective doses, which allows achieving the more pronounced corrective effect at a minimal cost. Such prophylactic strategy based on achieving a synergetic effect owing to the mutual potentiation of substances of different physico-chemical nature, is noted for its a priori universality and enables to significantly reduce the negative effect of broad-spectrum xenobiotics.

Under given circumstances, in this experiment we have studied the possibility of using in the compound feed for broiler chickens of three modifications of "Ecofiltrum" developed by specialists of the LLC "Leksir" and containing hydrolyzed lignin and prebiotic at variable ratios. To this end, the chickens of control group 1 were fed the mycotoxin-free basic ration (BR1) with nutrition parameters set as recommended by feeding standards of VNITIP (Table 3). The second control group (group 2) received the analogous ration (BR2) which was contaminated by mycotoxins in amounts causing a noticeable reduction of productivity, namely, Aflatoxin B₁ - 0.087 mg/kg [3.4 MRL], Ochratoxin A - 0.16 mg/kg [3.2 MRL], T-2-mycotoxin - 0.39 mg/kg [3.9 MRL] and Fumonisin B₁ - 11.5 mg/kg [2.3 MRL]. The indicated types of secondary metabolites of micromycetes most often found in the majority of poultry farms of central Russia, were introduced in the compound broiler feed as a corn-based fungal biomass containing the toxigenic strains of 4 producer mould strains (*Fusarium graminearum, F. sporotrichiella, F. moniliforme and Aspergillus flavus*) along with their toxic waste products, and also by inclusion to the feed mixture of laboratory-isolated and purified extracts of corresponding mycotoxins. Besides the indicated producer strains, the ration of experimental birds did not contain any traceable amounts of any other xenobiotics.

**Table 3. Design of experiment 2**

| Groups | Feeding particulars |
|---|---|
| *To investigate the possibility of "Ecofiltrum" use for reduction of negative impact on poultry of the feedstuff containing toxic fungal moulds* | |
| 1. Control (C1) | BASIC RATION WITHOUT MYCOTOXINS AND WITH NUTRITION PARAMETERS CORRESPONDING TO THE RECOMMENDED STANDARDS OF VNITIP [BR1] |
| 2. Control (C2) | BASIC RATION WITH NUTRITION PARAMETERS CORRESPONDING TO THE RECOMMENDED STANDARDS OF VNITIP, PLUS THE MYCOTOXIN MIXTURE: AFLATOXIN B₁ [0.087 MG/KG], T-2-MYCOTOXIN [0.39 MG/KG], OKHRATOXIN A [0.16 MG/KG] AND FUMONISIN B₁ [11.5 MG/KG] - [BR2] |
| 3. Experimental | BR2 + (1.0 KG/T LIGNIN + 0.5 KG/T LACTULOSE) - EF-1 |
| 4. Experimental | BR2 + (2.5 KG/T LIGNIN + 0.5 KG/T LACTULOSE) - EF-2 |
| 5. Experimental | BR2 + (4.0 KG/T LIGNIN + 0.5 KG/T LACTULOSE) - EF-3 |
| 6. Experimental | BR2 + (4.0 KG/T LIGNIN + 1.0 KG/T |
| | FRUCTOOLIGOSACCHARIDES) |
| 7. Experimental | BR2 + 4.0 KG/T POLYPHEPAN |

The mycotoxins contaminated compound broiler feed for chickens of experimental groups 3 - 5 included various amounts of lignin (1.0 kg/t, 2.5 kg/t and 4.0 kg/t) in combination with lactulose (0.5 kg/t). These are the component varieties of commercial preparation "Ecofiltrum®" in which the ratio of ingredients was 2:1, 5:1 and 8:1, respectively. It should be noted that the need of comprehensive investigation of prophylactic properties of either modification used for prophylaxis and treatment requires its objective comparison with the already existing counterparts. For this reason in the course of this experiment two experimental groups were additionally formed, in one of which (group 6) the maximum amount of hydrolyzed lignin (4.0 kg/t) was used in combination with another prebiotic preparation based on fructooligosaccharides (FOS) in the quantity of 1,0 kg/t of feed. Experimental broilers of group 7 received the feed supplemented with an aliquote of another analogue of hydrolyzed lignin, "Polyphepan®" (manufactured by CJSC "Saintech")¹ which differs from the above mentioned preparation by a higher moisture content (up to 30%), and absence of any other biologically active additives. All treatment and prophylaxis preparations were introduced in the compound broiler feed at the stage of feed preparation by adding compounds to the basic ration via a stepwise mixing.

In the end of the period of chicken raising the digestion trial was held in accordance to «Guidance for conducting scientific researches on feeding of poultry». The purpose of digestion trial was to study digestion and usage of nutrients in the ration, and also to determine the excretion of mycotoxins and vitamins. For this purpose 3 male broiler chickens of 5-week age were collected from each group. The digestion trial was divided into two periods: the preliminary period lasted for 5 days, and the balance period lasted for 3 days. The quantity and chemical composition of consumed feed and excreted dung of each chicken was analyzed individually.

Upon completion of this scientific experiment physiology-biochemical tests were carried out. To conduct scheduled analyses, 8 36-day old birds (4 male and 4 female birds) collected from each group were decapitated in accordance to the "Guidance for conducting studies in physiology and biochemistry". Samples of blood, chymus and liver were collected during chicken slaughtering.
¹The required amount of "Polyphepan" for this scientific and applied experiment was provided by the LLC "Leksir".

The biochemical tests were carried out in a certified laboratory of mycotoxicology (Certificate Nº ROSS RU.0001.21PCh64) and the research center of GNU VNITIP.

### Recorded parameters

### Zootechnical:

- survival of chicken (by daily recording bird mortality and finding out the causes of mortality);
- body weight at the age of one day, three- and five-weeks by individual weighing of all chicken;
- average daily feed consumption by daily recording of the parameter in each group;
- average daily weight gain and feed consumption per 1 kg of the body weight of broiler chickens (feed conversion rate) (by calculation at the end of growth period);
- European broiler productivity index (EBPI) by using calculations according to the equation: {(body weight [kg] x survival [%]) : (period of feeding [days] x feed conversion rate [kg/kg])} x 100%.

### Physiological and biochemical:

digestibility and assimilation of nutrients, and excretion of mycotoxins present in compound broiler feed:
   - total nitrogen in feed and manure - by Kjeldahl method;
   - raw protein in feedstuff - by calculations (N x 6.25);
   - raw protein in manure - by Dyakov's method (N x 6.25);
   - raw fat - by Sokslet method;
   - raw fiber in feed and manure - by acid-alkaline hydrolysis according to Hennenberg-Shtoman;
   - fat-soluble vitamins A and E (retinol and α-tocoferol) - using the method of microcolumn normal-phase high pressure liquid chromatography with UV-detection (B.D. Kalnitsky);
   - water-soluble vitamin B₂ (riboflavin) - fluorescence method;
   - mycotoxins in the feedstuff, manure and liver - using the method of solid-phase competitive immuno enzyme assay (IEA) (G.P. Kononenko);
   - total plasma protein - by biuret assay;
   - nucleic acids (DNA + RNA) in the liver - method of I.P. Simakov;
   - plasma glucose - glucose oxidase method;
   - total plasma cholesterol - by Ilk's method;
   - pyruvic acid in the liver - method of B.I Antonov;
   - hematological parameters (hemoglobin by cyanmethemoglobin method, erythrocytes by cell counting in hemocytometer, hematocrit by blood centrifugation in the capillaries);
   - Content of Lactobacilli (Lactobacillus and Bifidobacterium) and Enterobacteriaceae in small and large bowel by bacterial culture on selective media;
   - Parameters of non-specific resistance (plasma lysozyme by turbidimetric assay, plasma bactericidal activity by the method of O.V. Smirnova and T.A. Kuzmina, titer of natural(normal) agglutinins (E. coli) by agglutination reaction using the method of V.M. Mityushnikov);
   - titrated acidity and lactic acid concentration in the colon content by the method of I. Gutmann & A.W. Wohlefeld.

The biometric analysis of experimental results was carried out using the variation statistics method (Student's t-test) with the aid of a personal computer and Microsoft Excel 9.0 software.

### 2.2. STUDY RESULTS AND DISCUSSION

The results of observation of birds' growing to the age of 5 weeks (Table 4) have shown that the survival of chicken in control group 1 which was fed the basic ration, was at a sufficiently high level (96 - 97%) and was not characterized by influence of feed factor (incubation abnormalities were the dominated reason of mortality). As a whole, this survival rate fully corresponds to specifications of cross-breed and is the evidence of "well-being" of veterinary sanitary conditions in poultry yard during the research.

Chronic mycotoxicosis caused by the introduction in compound chicken feed of two fusarium (T-2 and FUM) and two aspergillus (OTA, AFLA) derivatives at the above given level (group 2), was characterized by significant reduction of the livestock survival (by 18.5%, P ≤ 0.02). The peak mortality was observed mostly in the initial period of growth (before day 21), and the mortality waste mostly comprised physically weak (dystrophic) chickens the majority of which demonstrated a full spectrum of anatomo-pathological changes characteristic for a given form of combined mycotoxicosis, namely, extensive caseous necrotic lesions of the oral cavity and crop, hyperemia and enlargement of the liver, dilatation of the bile ducts and gallbladder, multiple hemorrhages in mucous membranes of gastrointestinal tract, dryness and induration of gastric mucosal surface, signs of lung edema, serous pericarditis, lesions and atrophy of spleen and thymus, white flake-like depositions on the kidneys, enteritis, cloacites, uratic diathesis. The survival of experimental birds which received all three types of "Ecofiltrum" tended to increase markedly in groups 3 - 5 by 5.3%, 10.6% and 13.2% (P ≤ 0.02), respectively, depending on the extent of feed supplementation with the compound, from 1.5 kg/t to 4.5 kg/t. In this respect it must be mentioned that the values of a given parameter in groups 5 and 6 also significantly exceeded (by 5.3% and 2.7%; P ≤ 0.10) those of their counterparts in group 7 fed with the same amounts of "Polyphepan" (4 kg/t), which was an obvious indication of practical expediency of complex therapy of mycotoxicoses as compared to a mere use of a single organic sorbent without lactulose (0.5 kg/t) or fructooligosaccharides (1.0 kg/t).

Study results also showed that the negative effect of toxic mould metabolites was manifested mostly in the inhibition of the growth rate of experimental birds which occurred against the background of a low-grade feedstuff and extremely high incidence of mortality. Thus, the body weight of broiler chickens of control group 2 (K2) (negative control group) appeared to be significantly lower both in the first (more than 18.7%, P ≤ 0.001) and second (by 17.4%, P ≤ 0.001) age periods. From the zootechnical standpoint, it fully conforms with the nature of changes typical for the associative and synergistic effects of the derivatives of fusarium and aspergillus moulds (aflatoxin B₁, T-2-mycotoxin, ochratoxin A and fumonisin B₁) on the chickens.

The average body weight of broiler chickens (calculated based on the normal sex ratio in the group, i.e., 50% males and 50% females, Table 2) receiving with feed all three types of antitoxic compounds, has increased significantly as compared to the control group 2 (K2), by 4.3 - 10.3% and 4.7 - 12.9% (P ≤ 0.10-0.001) for the 3-week and 5-week old birds, respectively. Nonetheless, the body weight of the total livestock was still lower by 17.5 - 13.1 % and 13.7 - 6.7% (P ≤ 0.10-0.001), respectively, as compared to control group 1 (feed without mycotoxins). However, a more pronounced positive dynamics was noted in the experimental group 5 in which broilers were fed "Ecofiltrum" at a maximum dose (4.5 kg/t) throughout the whole period of growth.

At the same time, it must be taken into account that statistically significant, as compared to the control group 2 (K2), and largely close values of body weight in chickens of groups 4 and 6 fed with mycotoxin-spiked compound broiler feed containing either [2.5 kg/t lignin + 0.5 kg/t lactulose] or [4 kg/t lignin + 1.0 kg/t FOS], indicate to a similar physiological activity of both preparations despite their different structure and composition.

Therefore, if we take the negative effect of mycotoxins manifested in 17.4% reduction of the chicken body weight, for 100%, then, the ability of studied compounds to compensate the negative effects of xenobiotics in each group can be tentatively balanced against the following values: group 3 - 21%, group 4 - 40%, group 5 - 61%, group 6 - 50% and group 7 - 47%. However, it must be taken into account that the body weight of chickens taken as a main criterion of efficacy of compounds tested at the backdrop of chronic mycotoxicoses, is very unequal owing to different age and sex of birds and their different stages of development in terms of their enzyme systems and protective-adaptive mechanisms. The similar calculations was conducted for the European broiler productivity index (EBPI) which is not just the objective parameter integrating the values of body weight and survival of chicken, but also the most valid and solicited growth estimate in the industrial poultry husbandry.

The results of this scientific and applied experiment have shown that at the end of the broiler growth period the pharmaceutical composition "Ecofiltrum" containing the maximum amount of lignin steadily remained at the lead position (61%). Despite the differences in survival and body weight among the survived chickens, the second place (in terms of the aggregate antitoxic effect) was demonstrably shared between the compounds fed to group 4 chickens [2.5 kg/t lignin + 0.5 kg/t lactulose] and group 6 chickens [4.0 kg/t lignin + 1.0 kg/t fructooligosaecharides] , i.e., 50 - 53%. The addition to broiler feed of a "Polyphepan" at maximum doses appeared to be less preferable (41%), and the type of the test compound with minimal lignin content, EF-1, duplicated the established dynamics and remained at the "bottom of the list" of the remedial measures preferable for industry-wide use (23%).

**Table 4. Zootechnical parameters of growth of broiler chickens fed on mycotoxin-spiked feed supplemented with "Ecofiltrum" compound**

| PARAMETERS | Groups | | | | | | |
|---|---|---|---|---|---|---|---|
| | 1 (C₁) | 2 (C2) | 3 | 4 | 5 | 6 | 7 |
| Survival over the growth period, % | 97.4 ± 2.6 | 78.9 ± 6.7³ | 84.2 ± 6.0² | 89.5 ± 5.0 | 92.1 ± 4.4 | 89.5 ± 5.0 | 86.8 ± 5.6¹ |
| Body weight of chickens of 3-week age, g | 803.3 ± 14.7 | 632.7 ± 13.3⁵ | 662.4 ± 18.5⁵ | 670.4 ± 11.8⁵ ₂ | 697.9 ± 9.9⁵ ₅ | 676.5 ± 8.5⁵₄ | 675.1 ± 15.5⁵₂ |
| Body weight of male chicken of 5-week age, g | 1778.1 ± 46.5 | 1447.2 ± 64.2⁵ | 1650.4 ± 44.8¹ ₃ | 1679.8 ± 52.6 ₄ | 1783.6 ± 36.7₅ | 1730.2 ± 51.7₄ | 1688.4 ± 54.34 |
| Body weight of female chicken of 5-week age, g | 2198.9 ± 55.5 | 1837.8 ± 56.4⁵ | 1781.6 t 64.0⁵ | 1880.6 ± 41.5⁵ | 1926.2 ± 26.5⁵ | 1901.0 ± 48.1⁵ | 1922.2 ± 53.0⁵ |
| Average body weight of chicken (50 *%* ♀ +50 % ♂), g | 1988.5 ± 51.0 | 1642.5 ± 60.3⁵ | 1716.0 ± 54.4⁵ | 1780.2 ± 47.1⁴ ₁ | 1854.9 ± 31.6² ₄ | 1815.6 ± 49.9³₂ | 1805.3 ± 53.7³₂ |
| Average daily weight gain, g/day | 55.6 | 45.7 | 47.8 | 49.7 | 51.8 | 50.7 | 50.4 |
| Total body weight gain in group, kg | 71.97 | 47.68 | 53.31 | 58.93 | 63.33 | 60.13 | 57.97 |
| European broiler productivity index, units | 311 | 169 | 201 | 241 | 256 | 244 | 227 |

| | | | | | | | |
|---|---|---|---|---|---|---|---|
| Note: here and onward, the digits in superscript and subscript ^{1....} ₅ designate the significance thresholds for P ≤ 0.10 ... P ≤ 0.001, the superscript means "in comparison with control" group 1, the subscript means "in comparison with control" group 2, asterisk (*) - in comparison with the group receiving a known "Polyphepan" compound [4 kg/t] (group 7). | | | | | | | |

With regard to the discouraging results obtained during broiler chickens' feeding with compound feed contaminated by the mould secondary metabolites and supplemented with EF-1, it should be noted that similar facts were reflected not only in the complex zootechnical parameters but were also confirmed upon visual examination. Broiler chickens fed on compound feed containing minimal amount of lignin plus 0.5 kg/t lactulose differed by appearance from the counterparts of other groups. These differences were particularly obvious in comparison with birds of the control group 1 (C₁). Thus, if the former (BR₂ or BR₂ + EF-1) demonstrated the unnatural wing position, ruffle feathers, depression, wobbliness, reluctant moving around the cage and frequent diarrheas, then the latter (BR₁ or BR₂ + EF-3) did not show any of the above symptoms. Conversely, chickens of this group were agile, had good appetite and clean feathers.

Explaining the obtained results by a simple deficiency of sorption material in the preparation EF-1, we should consider that the biologically active substances contained in the same preparation can exert diverse effects on the animal body where, depending on their combined activity, the effect is either synergistic or antagonistic. As in the course of our experiment we used several types of "Ecofiltrum" (with different ratio of components), the successful experimental design made it possible to evaluate the effect of each ingredient on its own, based on the aggregate effect of complex preparation.

Using a two-factor mathematical analysis we found that when 3-week old chickens were fed with all types of "Ecofiltrum", then, every kilogram of hydrolyzed lignin (X) introduced in the compound feed promoted the recovery of the average daily weight gain, on average, by 5%, while every kilogram of prebiotic (U) contained in the preparation indirectly increased poultry production by 25%. Therefore, the recovery of body weight of broiler chickens manifested in the reduction of its values from 803 g to 634 g (difference between control groups which is tentatively taken for 100%), can be described by the equation: [K (%) = 5X + 25U].

However, at the end of production period, and at the backdrop of steady effect of lactulose, the positive effect of enterosorbent increased by at least 2-fold, indicating that hydrolyzed lignin appeared to be more effective in protecting 5-week old chickens from the harmful effect of high concentrations of xenobiotics contained in the feedstuff [C (%) = 10X + 25U]. Based on the above stated, it can be noted that despite in every type of "Ecofiltrum" lactulose appeared to be the more active component, the quantitative predominance of lignin (1.0 kg/t ... 4.0 kg/t) is a major factor in the more pronounced positive effect. Such data not only indicate to the desirability of rational combination of these components but also may warrant a differential approach to prophylaxis of mycotoxicoses based on the age of poultry and with regard to the economic aspects of poultry husbandry.

Based on the already considered parameters it is worth mentioning that the comparatively lower values of the body weight, survival of chicken and, as a consequence, aggregate weight gain in the groups fed with different amounts of studied preparations against the background of the mycotoxin contaminated compound feed, attest to that their effect was not sufficient for complete reduction of toxic effect caused by the introduction of large amounts of xenobiotics in the feedstuff (Σₜₒₓᵢₙ = 12.8 MRL). However, a significant and reliable increase of poultry body weight (during both age periods), as well as significant reduction of mortality of chicken as compared to the control group 2 (C2), are a the confirmation of the ability of all preparations with high lignin content to promote excretion of toxic agents from the body and minimize the combined form of chronic mycotoxicoses. This study provided the evidence that such combined preparations can be used for prophylaxis and treatment of mycotoxicoses throughout the whole period of broiler chicken production.

The analysis of similar experimental studies on mycotoxicology shows that in the majority of cases the relative values of body weight vary widely, and, depending on the background content of mycotoxins in the feedstuff, may have a similar range of fluctuations (%) against the control values. However, only the comparison of the data obtained with baseline toxicity enable to yield correct insight into the efficacy of given preparation. Our study was dealing with feeding a highly toxic feedstuff, and the toxicity was alleviated by correct treatment which eventually resulted in significant increase of productivity. Therefore, we can confidently forecast that with the majority of industrial rations containing a rather unremarkable amount of fungal secondary metabolite producers (1-5 MRL) the use of indicated pharmaceutical combinations can be even more effective. For this reason it seems feasible to reduce the amount of preparation added to the feedstuff by 1.5 - 3-fold without changing the recipe.

A similar relationship, which confirmed the correctness of our previous conclusions, was found upon analyzing a daily feed consumption pattern (Table 5). The materials collected in the course of experiment have also shown that the debilitating effects of mycotoxins in growing broiler chickens was particularly noticeable in control group 2 where, as a result of depressive state (inclusive of appetite loss) and mucous membrane lesions, chickens exhibited not only the absolute reduction of quantity of feed consumed by one bird over the period of its growth, but also the diminished effectiveness of feed biotransformation (FCR - 2.19 against 1.78 kg/kg in control group 1 (C1)). All this reflected the intense metabolic situation caused by neutralization of xenobiotics in organism, accompanied by high unproductive expenses of endogenous energy of chickens.

Under these circumstances, it is quite indicative that the relative feed usage, which increased during broiler chicken consumption of large quantities of mycotoxins, was significantly reduced (by more than 7-14%) after the ration has been supplemented with the studied pharmaceutical composition, that apparently resulted in normalization of metabolic processes in broiler chickens. As feed conversion rate is one of the most important parameters of production effectiveness, these data presents not only the economic significance but also enable to directly evaluate functional body response to any given amount of invading foreign agents, and thereby obtain an indirect evidence of effectiveness of therapeutic and prophylactic preparations. Therefore, the increased efficiency of nutrients use from contaminated feed mixture under the influence of tested - preparations can be associated with its positive effects stipulated drugs ability to weaken negative influence of xenobiotics on the growing organism of poultry.

At the same time, it is worth noting that after supplementation of ration with high lignin content preparations (groups 5 and 6) the feed conversion effectiveness appeared to be somewhat below the forecasted level derived from the analysis of positive dynamics in the neighboring groups (groups 2, 3, 4). Apparently, the explanation for the less pronounced stabilization of biological and production parameters can be found in the digestion studies, evidencing the ability of broad-spectrum sorbent used in high dosages at the high level of mycotoxins in feed to intensively excrete from the body not only toxic but also a number of nutrients and biologically active substances, and eventually diminish the general positive effect of the preparation.

The results of the digestion experiments (Table 5) have detailed the nature of changes found at the presence of mycotoxin contaminated feed. Thus, the presence in the compound broiler feed of secondary mould metabolites caused a significant reduction of parameters of nutrient digestion by 5.8 - 8.7% (P ≤ 0.001). The greatest changes were observed in crude protein, carbonhydrate components, crude fat and crude fiber. These effects in combination caused a decrease in digestibility of dry matter and gross energy use of the feed (by 6.6 %, P ≤ 0.001). The effectiveness of nitrogen retention and deposition promoting broiler chicken weight gain was reduced by more than 9% (P ≤ 0.001). A significant fraction of incoming amino acids had undergone the irreversible catabolic changes and was excreted from the body in the form of non-protein nitrogen (uric acid) at that. All these changes, along with the actual reduction of feed consumption, brought about both the inefficient feed utilization and reduced growth rate of chickens over the production period, and zootechnical results of poultry breeding in the control group 2 (C2) which received a total of 12.8 MRL of mycotoxins.

While analyzing the values of digestibility and use of nutrients in experimental groups 3 - 7, it can be confidently concluded that all tested preparations brought positive results. As a result of their administration in chickens, the digestibility of dry feed increased, on average, by 3.0 - 6.2% (P ≤ 0.05 - 0.001). The gross energy of intake also increased, including such energy- intensive components as crude fat (by 1.5 - 3.7, P ≤ 0.10 - 0.001) and the carbohydrate fraction of the ration (NFES) (by 4.4 - 8.6%, P ≤ 0.05 - 0.001), which is only possible if toxic agents are at least partially neutralized. Therefore, the combinations of sorbents and prebiotics fully reflect their ability to minimize the negative consequences, and confirms the synergistic effect in the prophylaxis of mycotoxicoses.

**Table 5. Effectiveness of feed consumption, digestibility of nutrients and biologically active substances, and deposition of vitamins in the liver of broiler chickens.**

| PARAMETERS | Groups | | | | | | |
|---|---|---|---|---|---|---|---|
| | 1 C1 | 2 C2 | 3 | 4 | 5 | 6 | 7 |
| Feed conversion rate*, kg/kg | 1.78 | 2.19 | 2.05 | 1.89 | 1.91 | 1.90 | 1.97 |

| Digestibility of nutrients | | | | | | | |
|---|---|---|---|---|---|---|---|
| Dry matter, % | 76.6 ± | 70.0 ± | 73.0 ± | 75.4 ± 1.6 | 76.1 ± 2.5 | 74.9 ± | 73.7 ± |
| | 1.5 | 2.7² | 0.9¹ | | | 3.2 | 0.8 |
| Crude protein, % | 84.9 ± 1.1 | 76.5 ± 1.8⁴ | 78.5 ± 2.1³ | 79.4 ± 0.4⁵ | 80.4 ± 1.4² | 78.9 ± 0.5⁵ | 79.6 ± 1.0⁴ |
| Crude fat, % | 74.7 ± 1.2 | 68.9 ± 0.5⁵ | 70.1 ± 2.3¹ | 72.4 ± 1.0¹₅ | 72.6 **±** 0.7 5 | 70.4 ± 0.641 | 70.0 ± 2.1¹ |
| Crude fiber, % | 18.1 ± 1.4 | 11.2 ± 0.7⁵ | 12.6 ± 2.1² | 15.5±1.9 2 | 15.9 ± 1.5 3 | 15.5 ± 0.8₄ | 14.7 ± 2.5 |
| NFES, % | 87.0 ± 1.0 | 79.9 ± 0.4⁵ | 84.3 ± 1.5 3 | 87.7 ± 3.0 2 | 88.5 ± 1.3 5 | 87.2 ± 1.7₅ | 84.9 ± 0.5¹ ₅ |

| Use of nutrients and biologically active substances | | | | | | | |
|---|---|---|---|---|---|---|---|
| Nitrogen (N), % | 40.6 ± 2.4 | 31.6 ± 1.5⁴ | 32.2 ± 1.5³ | 36.4 ± 1.8₁ | 35.5 ± 2.1 | 34.0 ± 0.9² | 33.2 ± 2.2² |
| Gross energy (Q), % | 76.4 ± 0.8 | 69.8 ± 1.7⁴ | 72.8 ± 0.7₄ | 75.2 ± 1.9₂ | 76.0 ± 1.1₄ | 74.6 ± 1.8₁ | 73.4 ± 1.4 |
| Vitamin E, % | 82.1 ± 1.0 | 72.6 ± 1.3⁵ | 75.3 ± 2.9² | 78.8 ± 3.7 | 76.6 ± 6.2 | 77.1 ± 3.1 | 69.8 ± 2.94 |
| Vitamin B₂, % | 70.1 ± | 52.3 ± 3.9⁴ | 58.3 ± 2.6² | 66.1 ± 4.8₂ | 60.7 ± 3.7 | 58.4 ± 4.6¹ | 66.1 ± 5.5 |

| Deposition of vitamins in the liver | | | | | | | |
|---|---|---|---|---|---|---|---|
| α - Tocoferol, µg/kg (vitamin E) | 17.52 ± 0.46 | 13.41 ± 2.07¹ | 16.23 ± 0.45¹ | 15.02± 1.21¹ | 14.79 ± 0.94³ | 14.45 ± 0.76⁴ | 15.57 ± 1.87 |
| Riboflavin, µg/kg (vitamin B₂) | 6.13 ± 0.92 | 4.02 ± 0.65¹ | 4.49 ± 0.84 | 4.59 ± 1.34 | 4.87 ± 0.76 | 5.01 ± 1.27 | 4.31 ± 1.11 |

| | | | | | | | |
|---|---|---|---|---|---|---|---|
| * Note: the parameter takes into account the body weight and survival of broiler chicken by the end of chicken growth period in each group. | | | | | | | |

However, upon introduction of all studied doses of "Ecofiltrum" and "Polyphepan" in the contaminated ration, the relative increase of digestibility of crude protein (by 2.0 - 3.9% and 3.1 %) and the efficiency of nitrogen retention in broilers (by 0.6 - 2.4% and 1.6%, P ≤ 0.05) taken as parameters reflecting body "involvement" in the biochemical confrontation with toxins, did not change significantly. As compared to the control group (C₁), these values remained among the lowest throughout the experiment (P ≤ 0.01 - 0.001). This enabled us to come to a definitive conclusion that none of the preparations used appeared to be sufficiently effective to neutralize mycotoxins completely. Chickens of all experimental groups were still lacking in required amounts of the plasticity material (nitrogen constituents). In order to compensate this shortage chickens consumed larger amounts of feedstuff, but had to spare the available energy of feed for neutralization of foreign substances in organism. Therefore, broiler chickens continued to be backward in amounts of growth and had low indexes of conversion of feed irrespective on inclusion of tested drugs into ration comparing with C1 control group.

It is necessary to mention, that the administration of all additives with sorbent compound into contaminated feed has their own biological features. On the one hand, possessing nonspecific mechanisms of a molecular sorption, sorbent can remove limiting and biologically active materials with high degree of polarity from digestive tract. In case of pathological process it can influence negatively to the severity of disease because of disturbances in animals' nutrition and attritions of internal reserves of a weakened organism forced to endogenously neutralize toxic agents. However, at the same time, by removal of toxicants and anticipation of development of nutritional toxemia, sorbents can - "soften" consequences of feeding poultry by substandard forage. The specified moments in mechanism of action of any active sorbent are exemplifying the antagonism intended for a positive side, the value of which (from the position of efficient production of animals) is always concerning scientists and experts. Thus, formed as a result of "intersection" of two - vectors of interaction, «the compromise point» characterizes efficiency of the usage of - sorbent for preventive purpose. Despite the fact that result is defined by a dose of administration of the preparation, its sorptive capacity, specificity of absorption of sorbate and even a physiological condition of animals, there are no cases that the result - productivity of poultry fed by contaminated feed would exceed the productivity of poultry fed by "pure" feed (mixed fodder without mycotoxins).

In this context, we must admit that the values of nutrient digestibility in experimental groups did not significantly vary compared to control group (C2). It is quite obvious that, along with the measurement errors (or so called "method precision"), the parameters studied were characterized by the comparatively low-power direct effect on the relative parameters of feed digestibility, which seemed to be offset by significant doses of sorbent. Looking at the results obtained, a disproportion between the increasing content of hydrolyzed lignin in the ration and physiological body response manifesting in the increased feed digestibility, is particularly noticeable. Thus, looking at the values of digestibility of dry matter (digestibility coefficient, DC) as a parameter reflecting the total consumption of nutrients, it is worth noting that although the DC value per every kilogram of added sorbent has increased in experimental groups, the overall trend was insignificantly downward. Thus, if the 1.5 kg increase of hydrolyzed lignin supplement in broiler feed (groups 3 and 4) promoted the increase in digestibility by 2.4% (or from 73.0% to 75.4%, respectively), then, in the next "1.5-kilogram" band (2.5 - 4.0 kg/t) the dynamic curve was losing its steepness making the difference as low as 0.7% (from 75.4% to 76.1%). Apparently, such differences can be explained by the sorbent's ability to bind and remove with feces a fraction of nutrients, thereby opening the way for compensatory consumption and non-economic waste of poultry feedstuffs. In this context, it should be underscored that the less active sorbent used with substandard ration, physically could not render such expressed positive effect, however, and its excessive quantity of an active agent obviously will not work positively for animal. Based on the above information, it is advisable to draw attention to both the presupposed reduction of preparation doses and increased background microbial fermentation; in which case the increasing hydrolysis of nutrients can minimize their counterproductive effect.

The other aspect of use of sorbent-based therapeutic and prophylactic compounds could be the enhanced removal of vitamins from the body, which (as all other polar low-molecular weight substances) are extremely susceptible to sorption. The importance of vitamins' balance study is determined by the fact that the latter are biologically active agents, which provide a normal course of physiological biochemical processes in organisms and have a big impact on metabolism. Domestic birds practically does not synthesize vitamins which expected deficiency is usually replenished by supplementing feed with vitamin and vitamin-mineral premixes. These are among the most costly components of poultry feed.

In the negative control group (C2) the efficacy of vitamin absorption has declined while their content in the liver remained at the lowest level throughout the experiment. Needless to say that the abnormal processes of cavitary digestion and mechanisms of absorption of biologically active substances by gastrointestinal mucous (primary deficiency), as well as the compensatory increased vitamin intake to inactivate xenobiotics (secondary deficiency), in combination with the effect of suppression of growth of intestinal normoflora which synthesizes group B vitamins, appeared to be the main causes of very low utilization and low deposition of biologically active substances present in broiler feed with a high content of toxic agents.

Notwithstanding the abovementioned arguments on total absorption, and in accordance with the more than once mentioned concept of sorbent-mediated improvement of poultry health, the amount of excreted fat-soluble (E) and water-soluble (B₂) vitamins has diminished significantly in experimental groups receiving sorbents (hydrolyzed lignin) as a supplement to mycotoxin-contaminated forages. Under these circumstances, vitamins were better absorbed, and their deposition in the liver was higher (on average, by 8-16% and 7-24%, P ≤ 0.10). It is a non-specific, yet quite informative test which complements the whole picture of negative changes which are due to test preparations, and the particulars of how the chicken body copes with these changes. Therefore, with such experimental design it does not seem possible to detect the excretion of excess vitamins. However, as in case with the digestibility of nutrients, groups treated with high concentrations of lignin (groups 5 - 7) demonstrated a marked trend towards the reduction of efficacy of their uptake, which was particularly clear manifested in a comparatively low deposition of vitamins (mostly vitamin E) in the liver, as compared to that in the neighboring broiler groups (groups 3 and 4).

By way of discussing the results obtained, it proper to note a more significant increase in fiber digestibility by 3.3 - 3.5% in all experimental groups receiving prebiotic preparations. It is known that fiber as a substrate practically is not hydrolyzed by avian gastrointestinal enzymes which digestive apparatus do not produce enzymes that destroy framework carbohydrates. Digestion of non-starchy biopolymers is the prerogative of symbiotic microflora of the intestine, quantity and functional activity of which of the experimental bird received 0,5 kg/t of lactulose and 1,0 kg/t of fructooligosaccharides, has appeared to be higher, but prevailed, apparently, at contemporaries which were fed by synthetic disaccharide of lactose. Therefore, the activation of microbiological processes turned out to be not just an important biological criterion of increased digestibility of nutrients but also a powerful correction factor affecting the productivity of animals suffering from combined forms of chronic mycotoxicosis (Table 5).

To summarize the data obtained in first part the course of this scientific and applied experiment, it can stated that the use of "Ecofiltrum" against the background of mycotoxicosis was characterized by positive dynamics from the standpoint of the industrial poultry breeding. The complex of experimentally obtained zootechnical parameters (survival, body weight and feed conversion rate) enabled to claim with certainty that the supplementation of broiler ration with high concentrations of the pharmaceutical combination has effectively reduced the negative effect of mycotoxins. That is why, the broilers from experimental groups significantly surpassed their counterparts from the control group 2 (C2), i.e., the experimental group broilers not only grew better and used feed more sparingly, but also effectively transformed feed nutrients into the body mass gain. However, the supplementation of forage contaminated with mixture of mycotoxins using the first pharmaceutical combination (EF-1) appeared to be less effective even in comparison with a simple native sorbent "Polyphepan". At the same time the maximum dose of the EF-3 combination still could not demonstrate the full anti-toxic potential of its comprising components because of the exorbitant sorption activity of lignin. Any specific concerns of this type must by no means be considered as a counterargument. We believe that it would be more practical to consider these arguments from the standpoint of additional (preventive) enrichment of feedstuff in case of very high micotoxin contents with vitamins, minerals and other biologically active components. Timely correction of rations by the specialists will enable to achieve a pronounced therapeutic effect.

Therefore, the pharmaceutical compositions based on the combination of high or middle lignin doses with lactulose and fructooligosaccharides, worked out by the LLC "Leksir" and named "Ecofiltrum" demonstrated positive effects on zootechnical parameters of avian growth in mycotoxicosis-affected livestock and can realistically compete with traditional feed additives possessing the antitoxic effects. The use of complex preparations for prophylaxis of mycotoxicoses is justified and is clearly demonstrated by the results of the experiment conducted.

Moving on to considering the other important parameters, it is worth noting that both - feed consumption and other forms of implication of somatic toxicosis in organisms of animals, and also the degree of expression of pathological process are directly defined by amount of mycotoxins, subjected to absorption. In this regard, the balance of foreign low-molecular weight substances subject to gastrointestinal absorption enables to additionally characterize the qualitative parameters of all different additives with the purpose of their effective use for prophylaxis of mycotoxicoses.

However, before discussion of the empirical data, we deem it appropriate to give the introductory information about the specifics of methods for determination of mycotoxins, as well as their metabolism in biological objects.

It is well known that many analytical techniques are used for determination of fungal metabolic products (mycotoxins), and one of such techniques is the enzyme immunoassay (EIA). This method strongly proved itself not only as an approved (arbitrary) method but also as a highly specific analytical technique. The EIA can detect not the entire multitude of all natural types of mycotoxins but only their certain (most common as a rule) chemotype, while the xenobiotic metabolites can not be detected by this method. At the same time, once in the body mycotoxins undergo profound transformation by the enzyme systems of the host (in the liver) and by normal intestinal microflora. The results of numerous fundamental studies have shown that xenobiotics are not only rapidly absorbed through mucous membrane but also rapidly metabolized (subject to detoxification or inactivation), and within 48 - 72 h nearly completely excreted with fecal masses in the form of transformed compounds. This means that the EIA assay can detect in the dung only a small fraction (5 - 20%) of the unchanged toxin originally present in the oral dose. However, with different prophylactic compounds these values may fluctuate upward or downward. Therefore, the presented parameters of "apparent toxin excretion" enable us to characterize the sorption properties of test compounds.

The results of balance experiment (Table 6) have shown that upon feeding of the mycotoxin-contaminated feed to broiler chickens of the control group 2 (C2), the majority of toxins have become available for absorption in the gastrointestinal tract (up to 82.1 % of T-2-mycotoxin, up to 74.6% of fumonisin B₁ and up to 86.6% of ochratoxin A), thereby bringing about marked toxic effect. Toxin content in the liver also remained sufficiently high. When the mycotoxin-contaminated forage was supplemented with "Polyphepan" (group 7), the degree of mycotoxin excretion was higher as compared to the control group 2 (C2), that predetermined sufficiently high production parameters of poultry breeding (Table 4). Thus, at a given level of sorbent addition to forage the evacuation of mycotoxins has increased significantly, namely, T-2-mycotoxin by 1.30-fold (P ≤ 0.05), ochratoxin by 1.77-fold (P ≤ 0.001) and fumonisin by 1.83-fold (P ≤ 0.001). In turn, the depression of an alimentary load of mycotoxins on organism has allowed to mobilize its protective power. As a result, there was a decrease of absolute content of toxic agent in the liver on 15-24 % (P ≤ 0, 02) which along with reduction of their intrusion into an organism, were more actively exposed to biochemical destruction and inactivation that has allowed not only to avoid occurrence of typical pathoanatomical changes, but also to restore substantially homeostatic function of the liver, including its detoxicant component.

Contrary to the expectations of high sorption activity of hydrolyzed lignin - prebiotic preparations, the apparent amounts of excreted mycotoxins (depending on the increasing quantity of their addition to forages) demonstrated a steady trend towards reduction by 18 - 40% (P ≤ 0.02-0.01) despite the presence in the preparation of the same amount of lignin (4.0 kg/t). However, these data is in contradiction with the data on xenobiotic levels in the liver - the main organ responsible for the barrier function against, and detoxification of foreign substances in animals. The results of study have shown that while in the control group 2 (C2) the mycotoxin concentrations remained at the maximum levels (up to 9.5 µg/kg for T-2-mycotoxin, 1.1 µg/kg for ochratoxin and 121.3 µg/kg for fumonisin) bringing about marked toxic effect, then, in the study groups administration of increasing amounts of "Ecofiltrum" caused a characteristic reduction of absolute content of secondary fungal metabolites in avian liver by 15 - 24% on average (all three mycotoxins), whereas such reduction after administration of Polyphepan was only 17%.

Such contraindication where are "lost" up to 10-30% of the daily dose ingested toxins could be explained by the quantitative parameters of gastrointestinal microflora. The results of study have shown (Table 6) that the presence of mycotoxins in forage had a significant impact on gut microbiota. Firstly, their presence in forage caused a 4-8-fold reduction of the titer of Enterobacteria, a vast group of bacteria including Escherichia coli, as well as of two main genera of lactobacteria, Lactobacilli and Bifidobacteria, represented by the parietal and cavitary microflora. Secondly, the use of poor quality feedstuff in poultry husbandry results in symptoms of chronic dysbacteriosis, that is, the change in the balance of microbial composition towards the increasing prevalence of putrefactive and conditionally-pathogenic forms over lactobacilli (from 1:28200 to 1:12300), as well as their increasing number in small bowel (2.6-fold, P ≤ 0.01), that at the backdrop of general birds weakening is fraught with the risk of occurrence of infectious diseases or acute intestinal infections.

**Table 6. "Ecofiltrum" effect on the balance of mycotoxins and intestinal microorganisms' content in broiler chickens**

| PARAMETERS | Groups | | | | | | |
|---|---|---|---|---|---|---|---|
| | 1 (C1) | 2 (C2) | 3 | 4 | 5 | 6 | 7 |
| Apparent excretion of mycotoxins from avian body | | | | | | | |
| T-2-mycotoxin, % | - - - | 28.8± 2.7 | 31.7± 0.6 | 32.7 ± 4.3 | 32.1 ± 1.9 | 32.8 ± 2.3 | 37.4 ± 2.6₂ |
| Ochratoxin A, % | - - - | 13.4 ± 1.6 | 17.5 ± 1.7 ₁ | 17.3 ± 1.6 | 16.2 ± 2.0 | 19.0 ± 1.7₂ | 23.7 ± 0.7₅ |
| Fumonisin B₁, % | - - - | 25.4 ± 1.3 | 35.1 ± 3.4₃ | 37.0 ± 5.0 | 31.6 ± 2.6 ₂ | 29.7± 4.6 | 46.6 ± 1.9₅ |

| Residual content of mycotoxins in dry liver of broiler chickens | | | | | | | |
|---|---|---|---|---|---|---|---|
| T-2-mycotoxin, µg/kg | - - - | 9.61 ± 0.88 | 8.76 ± 0.27 | 8.05 ± 0.71 | 7.77 ± 0.57₁ | 7.81 ± 0.27₁ | 8.15 ± 0.36 |
| Ochratoxin A, µg/kg | - - - | 1.08 ± 0.05 | 0.90 ± 0.07 ₁ | 0.81 ± 0.05₄ | No data available | 0.76 ± 0.12₂ | 0.82 ± 0.08₃ |
| Fumonisin B₁, µg/kg | - - - | 121.35 ± 3.93 | 109.73 ± 3.68₃ | 102.52 ± 4.64₄ | 95.12 ± 6.47₄ | 98.74 ± 5.25₄ | 103.87 ± 4.55₃ |

| Microbiological parameters | | | | | | | |
|---|---|---|---|---|---|---|---|
| Small bowel (chymus) | | | | | | | |
| Enterobacteriaceae, 1g | 2.9 ± 0.2 | 3.3 ± 0.1¹ | 3.1 ± 0.1 | 3.2 ± 0.1 | 3.2 ± 0.1 | 3.1 ± 0.1 | 3.2 ± 0.2 |

| Large bowel (content) | | | | | | | |
|---|---|---|---|---|---|---|---|
| Enterobacteriaceae, 1g | 5.88 ± 0.14 | 5.32 ± 0.11⁴ | 5,45 ± 0.09² | 5.51 ± 0.01³ | 5.61 ± 0.08₂ | 5.97 ± 0.11₂ | 5.57 ± 0.08₂ |
| Lactobacillus, 1g | 7.42 ± 0.05 | 6.56 ± 0.23⁴ | 7.19 ± 0.07³₃ | 7.24 ± 0.11₃ | 7.49 ± 0.29₂ | 7.34 ± 0.14₃ | 7.08 ± 0.16¹₁ |
| Bifidobacterium, 1g | 10.33 ± 0.07 | 9.41 ± 0.05⁵ | 9.87 ± 0.12⁴₄ | 10.03 ± 0.15¹₄ | 10.19 ± 0.12₄ | 10.23 ± 0.14₅ | 9.83 ± 0.11⁴₄ |
| Total ammonia, mg% | 6.5 ± 0.4 | 9.3 ± 0.5⁵ | 8.1 ± 0.5⁵ | 7.5 ± 0.4¹₃ | 7.1 ± 0.8₂ | 8.1 ± 0.3⁴₁ | 7.8 ± 0.5¹₂ |
| Lactic acid, mg/g | 2.7 ± 0.1 | 2.5 ± 0.1¹ | 2.7 ± 0.1₁ | 3.0 ± 0.1²₅ | 3.3 ± 0.1⁵₅ | 3.3 ± 0.1⁵₅ | 3.1 ± 0.1³₅ |
| Acidity titrated (acetate), mg/g | 9.6 ± 0.7 | 7.7 ± 1.0 | 9.1 ± 0.8 | 9.0 ± 0.6 | 8.1 ± 0.2¹ | 8.7 ± 1.7 | 8.4 ± 1.0 |

While analyzing these tables, it can be noted that the administration of the preparation "Polyphepan" promoted a 1.8 - 3.3-fold increase of saprophyte bacteria. Nevertheless, the putrefactive microflora of Enterobacteriaceae (which main representative is Escherichia coli) still remained predominant causing an intense production of ammonia in the large bowel (1.4-fold higher, P ≤ 0.01). All this did not promote the maximum unmasking of positive potential of monotherapeutic measures because of the increased negative load of toxic end-products of protein degradation on the growing chicken body. In this regard the efficacy of "Ecofiltrum" was higher as the product containing lactose (normally unavailable in the avian body) acted like a growth promoter for lactobacteria and bifidobacteria which total number has increased 3 - 9-fold (P ≤ 0.01) as compared to the control group 2 (C2). Such changes lead to the formation of normal biocenoses via the intensive gut colonization by normal species-specific symbiotic microorganisms (1:38000) and prevention (via the competitive mechanism) of intestinal colonization by conditionally-pathogenic bacteria and increased resistance to alimentary infection and to the negative effect of feed.

It should be noted that depending on the increasing lignin content (from 1.0 kg/t to 4 kg/t), when lactulose remained constant (0.5 kg/t) in the mycotoxin contaminated feedstuff, there was a more significant accumulation of lactic acid (the end-product of fermenting microflora metabolism) and organic acids in the content of the caecum. Apparently, the use of sorbent creates more comfortable conditions owing to binding of the bulk of free toxins, while a fraction of lactulose is passed to the lower parts of gastrointestinal tract where its more effective use by the intestinal lactic flora formed the basis of synergistic action between the two components of complex preparation. At the same time, despite a similar content of lactate and titrated acidity in the same parts of gastrointestinal tract of broiler chickens receiving fructooligosaccharides, the ratio of lactic and putrefactive microflora appeared to be 1:18200 which, from the microbiological standpoint, can not just be explained by the less selective action of FOS but also enable to reveal practical advantages of given prebiotic preparations.

In conclusion of this sub-section, it is necessary to mention, that the specificity of application of prebiotics in case of mycotoxicosis is in many respects defined by improvement of the trophic status of microflora which, being in the digestive tract of a bird, has survived and has got ability to distract toxic agents. Classical studies have shown that the metabolic process in microbial organelles, such as oxidation or conjugation, are in many instances identical to enzymatic reactions matching those observed during phase II of the detoxification process in higher animals. Thus, the principal similarity of biochemical processes promotes either death of microorganisms owing to their poisoning with toxifying xenobiotics (products of intermediate metabolism of original xenobiotics) or survival of some species posseses the ability to metabolize foreign toxic substances into the non-toxic compounds. In the course of their intensive growth symbiotic microorganisms tend to interact with foreign substances in GIT thereby reducing their alimentary load on host organism and minimizing the effect of mycotoxins, making the outlook for application of "Ecofiltrum" for this purpose promising. However, for more successful conduction of health promotion program in case of feeding by substandard forages and constant inclusion of this complex action preparation it is advisable to periodically update cultural properties of intestinal microflora by a one-off testing of the poly-strain prebiotic preparations containing physiologically modified assortment of microorganisms.

The parameters of protein metabolism and a series of total metabolism parameters are a sufficiently informative indication of the effectiveness of use of structural plasticity material in the avian body affected by the study factors. The results of study (Table 7) have shown that in the control group 2 (C2) (ration contaminated by mycotoxins) the total content of free amino acids in broilers' blood was significantly higher (by 40%, P ≤ 0.001) against that of their counterparts in the control group 1 (C1), but the serum protein content appeared to be reduced by 13.7% (P ≤ 0.001). The analysis of the data obtained allows the conclusion that chronic mycotoxicosis was accompanied by the diminished intensity of biosynthetic processes, when the most amino acids in the body of experimental birds were not assimilated but instead accumulated in the blood as the leftover products.

The experimental groups of chickens (despite having marked signs of a chronic process) demonstrated a characteristic and significant increase of both plasma protein (by 2.5 - 5.4%) and nucleic acids in metabolically active tissues (by 10 - 27%) (P ≤ 0.10 - 0.02). However, the reduction of free amino acids (by 4 - 15%; P ≤ 0.05 - 0.01) was an unequivocal indication of stimulation of protein biosynthesis and elimination of gross toxic effect caused by feeding poor quality forages to chickens.

It is well known that nitric oxide metabolism is the most susceptible metabolic process when the body is affected by toxic metabolites of the most common mould fungi. Proteins are involved in a large variety of physiological functions, and the inhibition of protein synthesis during mycotoxicoses leads to drastic changes in protein functions and their linked enzyme systems. As a result, the intensity of the important protein-regulated vital activities is declining, and the ability of the body to resist the unfavorable external and internal factors is changing as well. Given that a significant fraction of hepatic proteins is represented by the enzymatically active proteins, the ability of the liver to produce any given protein, even to a limited extent, determines the whole metabolic processes, type of compensatory changes and structure and function of tissues. The abnormal protein metabolism is not only the most frequently occurring and hardly avoidable component of toxic pathology but in many cases can even aggravate the pathological process at that.

However, the addition of sorbent to chickens' ration enabled to significantly (by 13-50%, 40% and 35%, respectively) block toxic effect caused by high doses of xenobiotics and activate the adaptive potential of the chicken organism. A positive effect of enterosorbents was indirectly manifested in accelerated synthesis of enzymatic proteins acting as a powerful regulator of enzyme reactions required for normalization of metabolism in farm animals and poultry, increase of their productivity and improvement of quality of farm produce.

The mycotoxin-contaminated forages also had a significant impact on other metabolic parameters. chickens in C2 group had reduced serum glucose (18.1%, P ≤ 0.02) and pyruvic acid in the liver (by 5.6%, P ≤ 0.05), but serum cholesterol and total lipids have increased by 31.2% (P ≤ 0.01) and 35.8% (P ≤ 0.01), respectively. Chickens demonstrated signs of hypochromic anemia, namely, hemoglobin saturation in single erythrocyte has dropped dramatically (by 21.5%, P ≤ 0.05), with a compensatory increase in red blood cell volume (by 17.3%, P ≤ 0.05), All this allowed the conclusion that the mycotoxin-contaminated feed led to significant inhibition of intermediate metabolism and less efficient oxidation of substrates required for the production of metabolic energy for avian growth.

In clinical practice such disturbances are particularly often found in the highly productive animals in case of their abnormal carbohydrate and lipid metabolism, which is linked to liver dysfunction and its inability to cope with physiological stress. Thus, the reduced glucose content attests not only to the deterioration of digestion of NFES contained in the forages (as demonstrated in the balance experiments, Table 3), but also to the deterioration of use of this energy source by avian peripheral tissues (glucose → glucose-6-phosphate → pyruvate). The link between the two parameters: "glucose - pyruvic acid" clearly indicates to the total reduction of intensity of oxidation-reduction processes in avian body during chronic mycotoxicosis, and during its first step, the glycolysis.

Whilst changes in lipid metabolism of broilers are important, there is a need to remember about close relationship between the lipid and carbohydrate metabolism.

It is well known that the fatty acids stored in the liver break down to generate acetyl-CoA. The acetic acid of CoA is combined with oxaloacetic acid and degrades in Krebs cycle to yield the end products (CO₂ and H₂O). However, if the breakdown of carbohydrates (glucose) does not yield sufficient amount of *pyruvic* acid (the precursor of oxaloacetic acid), then, the oxidation of fatty acids goes via formation of ketone bodies which are oxidized in peripheral tissues better that fat. As the liver is the exclusive site for ketone bodies formation, then, fat mobilization from the peripheral depots provokes fat infiltration. On the other hand, the sources of endogenous lipids are ketogenic substrates (such as sugars, acetic acid, leucine, valine, etc.). Therefore, when the activity of dehydrogenases in Krebs cycle is inhibited, this creates favorable conditions for their synthesis and conversion to acetyl-CoA to eventually yield lipids and cholesterol. Should the oxidation processes be inhibited, fat and re-synthesized triglycerides "supplied" with blood do not undergo degradation, oxidation or excretion but instead are deposited in tissues for long-term storage and circulate in blood. Therefore, when the forages are contaminated with mycotoxins, the metabolic effect manifests itself in carbohydrate and protein conversion to different classes of lipids leading the energy balance from carbohydrate oxidation to intensive lipid degradation.

The biochemical investigations have confirmed the results of the invasive visualization. Upon the autopsy of broilers from the control group 2 (C2) there were pathological changes of the color, histological structure and architectonics of the liver characteristic for *fatty infiltration* of the liver. The liver was yellow or reddish brown, of flabby consistence and with hemorrhages on its ventral surface. Histological picture of fatty liver dystrophy was characterized by degenerative changes of hepatocytes and marked expansion of fat vacuoles. This resulted in increased liver size and its flabby consistence, the liver could be easily ruptured, showed excess fat depositions and altered color. Fatty infiltration of the liver apparently led to its impaired function for the total body metabolism, and this had a negative impact on poultry productivity and liveability during the growth period. At the same time, the use of studied pharmaceutical preparations enabled not only to prevent the appearance of characteristic anatomo-pathological alterations (i.e., fatty hepatosis) but to restore nearly completely the homeostatic function of the liver inclusive of its detoxification function.

Determination of similar parameters in experimental broiler chickens receiving the pharmaceutical preparations being studied, against the background of mycotoxin-contaminated feedstuff, indicated to general normalization of physiological and biochemical processes during which the peripheral tissues received adequate supply of oxygen, and the mechanisms of hemoglobin synthesis and transport were normal. The quantitative values of these parameters returned back to physiological norms and practically did not differ from those in their counterparts in the control group 1 (ration without mycotoxins). Therefore, the studied pharmaceutical preparations were instrumental in overcoming negative changes of carbohydrate and lipid metabolism and energy balance in poultry (by 50 - 70%).

The issue of immunity is another global aspect of the problem when producers are forced to feed poultry with low quality forages. Modern species crosses of birds are known for their high demand for environmental conditions during breeding. Nowadays birds grow much faster and require less feed per unit of body weight gain than it was even 10 years ago. However, high productivity broilers and layers often have lower resistance. In the conditions of increased microbial antigen load and enclosed poultry house space this factor is [] very important. While considering the immunosuppression issue, it should be noted that the mycotoxin-containing forages (along with other immunosuppressing agents circulating in the farm) can act as the immunosuppressive agents by contributing to the inhibition of protein synthesis and suppression of lymphoid organ development, and suppressing cellular and humoral immunity, as well as the non-specific immunity factors.

**Table 7. "Ecofiltrum" effect on certain metabolic parameters, protein metabolism parameters and resistance in broiler chickens**

| PARAMETERS | Groups | | | | | | |
|---|---|---|---|---|---|---|---|
| | 1 (C₁) | 2 (C2) | 3 | 4 | 5 | 6 | 7 |
| General biological parameters | | | | | | | |
| Plasma proteins, g/l | 39.3 ± 12 | 33.9 ± 0.1⁵ | 34.3 ± 1.4³ | 34.8 ± 1.4² | 35.7 ± 2.1 | 35.8 ± 1.2¹ | 37.0 ± 2.7 |
| Free amino acids (total), mg% | 63.2 ± 1.4 | 90.6 ± 4.1⁵ | 86.9 ± 3.3⁵ | 81.6 ± 1.7⁵₁ | 77.1 ± 2.7⁵₃ | 82.0 ± 2.0⁵₁ | 81.9 ± 1.1⁵₁ |
| Raw liver nucleic acids, % | 1.76 ± 0.18 | 1.12 ± 0.09⁴ | 1.23 ± 0.05³ | 1.32 ± 0.10² | 1.56 ± 0.11 ₄ | 1.48 ± 0.10₃ | 1.22 ± 0.07³ |
| Total serum lipids,% | 0.81 ± 0.05 | 1.10 ± 0.04⁴ | 0.86 ± 0.12₂ | 0.82 ± 0.09₄ | 0.93 ± 0.04₄ | 0.80 ± 0.04₅ | 0.87 ± 0.06₄ |
| Cholesterol, mM/L | 3.2 ± 0.1 | 4.2 ± 0.3⁴ | 3.8 ± 0.1⁵ | 3.8 ± 0.2³ | 3.4 ± 0.2₂ | 3.6 ± 0.1³₁ | 3.9 ± 0.3² |
| Glucose, mM/L | 8.3 ± 0.5 | 6.8 ± 0.2³ | 7.8 ± 0.3₃ | 7.8 ± 1.1 | 9.8 ± 2.2 | 7.9 ± 0.4₂ | 6.9 ± 1.3 |
| Liver pyruvate, mg/g | 14.2 ± 0.3 | 13.4 ± 0.2² | 13.9 ± 0.3 | 14.3 ± 0.3₂ | 14.5 ± 0.1₅ | 13.9 ± 0.1₂ | 13.8 ± 0.1₁ |
| Hemoglobin content of red blood cells, % | 25.1 ± 0.7 | 19.7 ± 1.9³ | 24.2 ± 0.9₂ | 24.4 ± 0.5₂ | 23.1 ± 0.8₁ | 22.6 ± 1.7 | 21.3 ± 1.3₂ |
| Mean corpuscular volume, fL | 76.9 ± 1,4 | 90.2 ± 5.4² | 82.4 ± 2.7¹ | 78.8 ± 6.1 | 78.3 ± 5.3 | 81.4 ± 4.9 | 77.5 ± 5.2 |

| Nonspecific resistance parameters | | | | | | | |
|---|---|---|---|---|---|---|---|
| Blood lysozyme, g/L | 4.7 ± 0.2 | 4.1 ± 0.1³ | 4.2 ± 0.5 | 4.4 ± 0.3 | 4.3 ± 0.8 | 4.2 ± 0.2¹ | 4.3 ± 0.4 |
| Serum bactericidal activity, % | 78.6 ± 1.9 | 67.2 ± 0.8⁵ | 70.5 ± 1.4⁴₁ | 69.3 ± 2.0⁴ | 71.7 ± 0.9⁴₄ | 72.8 ± 3.6 | 70.2 ± 2.1₃ |
| Total immune proteins, CST units | 16.8 ± 1.2 | 12.8 ± 1.0¹ | 15.0 ± 1.6 | 14.7 ± 0.8 | 14.8 ± 0.7 | 15.2 ± 2.2 | 14.5 ± 0.9 |
| Normal | 322 ± | 249 ± | 268 ± 35 | 277 ± 74 | 276 ± 65 | 328 ± | 303 ± |
| antibody titer (RNA₅₀), 1:X, units | 47 | 72 | | | | 82 | 91.4 |

The results of study have shown that broiler chickens receiving rations with inclusion of lignin in high doses and prebiotics (groups 4-6) are notable for higher parameters of non-specific resistance as compared to their counterparts from other groups (Table 7). Thus, the activity of several antimicrobial humoral factors, namely, serum lysozyme and bactericidal activities, as well as the anti-E. coli agglutinin titer, showed a clear upward trend, on average, by 30 - 42% (P ≤ 0.10 - 0.01), that, along with the higher poultry production, contributed to better survival of broilers over their breeding period (Table 2). Apparently, the explanation for this can be found in the normalization of protein metabolism as well as in the distinctive features of symbiotic microflora which, owing to its high concentration, can exert the adjuvant effect on intestinal macrophages and, via the haphazard translocation into parenchymatous organs, stimulate immune system, enhance protective mechanisms of intestinal mucosa and protect the immune system from damage on the whole. Taking into account a tense epizootic situation at poultry husbandries, this could be an utterly and completely positive point for justification of industrial use of "Ecofiltrum" for treatment in case of mycotoxin contaminated forages.

Based on the results of study conducted it is possible to conclude that these experimental data convincingly demonstrate that "Ecofiltrum" developed by the LLC "Lexir" is a sufficiently effective antitoxic agent. However, under extremely high toxicity level only those combinations of hydrolyzed lignin and lactulose appeared to be most efficient wherein the enterosorbent was used at 2.5 - 4.0 kg/t, which allowed mobilization of body defense system of birds suffering from chronic combined mycotoxicoses with no marked negative consequences. In this regard, a certain superiority of zootechnical parameters in experimental groups over those in groups where the native hydrolyzed lignin was used, seems to be reasonable as "Ecofiltrum" exerts a complex physiological effect on metabolism and bodily functions. This highly effective enterosorbent promotes active excretion of toxic components while lactulose augments the role of symbiotic microflora in utilization of xenobiotics. This is characteristic for specialist therapeutic and preventive health products, and enables to consider "Ecofiltrum" as one of the main factors that contribute to homeostasis. It should be noted that the trichothecene mycotoxins used in these model experiments are the non-polar compounds and extremely poorly excreted from the body when the majority of currently available sorbent preparations are used. However, this complex preparation used for detoxification purposes clearly demonstrated a pronounced protective effect which enabled to obtain additional benefits during breeding of broiler chickens fed on the non-polar mycotoxin contaminated rations.

Calculation of economic indicators (Table 8), while using three types of lignin - lactulose combinations (group 3 - 5), one lignin - FOS combination (group 6) and "Polyphepan" (group 7) in broiler feedstuff, has shown that at the average forage market price of 11.80 rub./kg, and the price of the above preparations from 230-140 to 200 and 90 rub./kg, respectively, their inclusion in the ration at the level of 1.5-4.5, 5.0 and 4.0 kg/t leads to insignificant appreciation of the forage cost (by 320-570, 510 and 310 rub./t).

Calculation of production costs has shown that "Ecofiltrum" use appeared to be economically sound at all doses (1.5 - 4.5 kg/t). Whilst the mycotoxin contamination of broiler chicken feedstuff brought about significant economic costs (about 13100 rub./1000 broilers), the use of these preparations (considering the current price trend for main forage resources) was instrumental in reducing the expenditure incurred by 15, 48 and 45%, or 1920, 6240 and 5830 rub./1000 broilers, respectively. Such a result was due exclusively to physiological effect of the given types of "Ecofiltrum" which were able to surpass the development of endogenous intoxication after the entry into the body of foreign substances of natural or anthropogenic origin, normalize the detoxification processes, non-specifically stimulate body resistance and thereby improve the performance indicators of chicken breeding.

Using the mathematical modeling and a parabolic function, one can suggest that the higher (over 4.0 kg/t) level of lignin addition to mycotoxin-contaminated forages will not promote the reduction of production costs during chicken meat production because of the increasing cost of pharmaceutical preparation and its high sorption ability. For the majority of broiler farms the most optimal variant from the economical standpoint (about 7000 rub./1000 broilers) will be the level not exceeding 2.8 - 3.2 kg/t provided the constant addition of lactulose (0.5 kg/t).

The cost-effectiveness analysis has shown that in case of a more costly but less efficient pharmaceutical preparation, i.e., the combination of lignin and FOS, it also demonstrated positive result which appeared to be quite comparable with that of "Polyphepan" in terms of compensation in case of unforeseen losses. Therefore, because the cost of the additional weight gain (which was due to the use of FOS) was practically equal to the cost of pharmaceutical preparation itself, the overall cost effectiveness of its use appeared to be very low (1.7-fold).

**Table 8. Cost- effectiveness analysis of "Ecofiltrum" use upon its inclusion into the mycotoxin-contaminated broiler forage**

| PARAMETERS | UNITS | GROUPS | | | | | | |
|---|---|---|---|---|---|---|---|---|
| | | 1 (C₁) | 2 (C2) | 3 | 4 | 5 | 6 | 7 |
| Nº of broilers taken for breeding | birds | 38 | 38 | 38 | 38 | 38 | 38 | 38 |
| Weight of a one day-old chicken | g | 42 | 42 | 42 | 42 | 42 | 42 | 42 |
| Period of broiler growth | days | 35 | 35 | 35 | 35 | 35 | 35 | 35 |
| Mortality | birds | 1 | 8 | 6 | 4 | 3 | 4 | 5 |
| Survival by the end of period of growth | % | 96.7 | 78.9 | 84.2 | 89.5 | 92.1 | 89.5 | 86.8 |
| Average weight of the one head by the end of period of growth | g | 1988.5 | 1642.5 | 1716.0 | 1780.2 | 1854.9 | 1815.6 | 1805.3 |
| Gross body weight | kg | 73.57 | 49.28 | 54.91 | 60.53 | 64.92 | 61.73 | 59.57 |
| Gross body weight gain | kg | 71.97 | 47.68 | 53.31 | 58.93 | 63.32 | 60.13 | 57.97 |
| Total feed consumption | kg | 128.11 | 104.42 | 109.29 | 121.99 | 120.94 | 114.25 | 114.20 |
| Incl. compound feed | kg | 128.11 | 104.42 | 109.13 | 121.63 | 120.40 | 113.68 | 113.75 |
| Incl. preparation | kg | - - - | - - - | 0.16 | 0.36 | 0.54 | 0.57 | 0.45 |
| Feed consumption per 1 kg of body weight gain | kg | 1.78 | 2.19 | 2.05 | 1.89 | 1.91 | 1.90 | 1.97 |
| Cost of 1 kg compound feed | Rub. | 11.80 | 11.80 | 11.80 | 11.80 | 11.80 | 11.80 | 11.80 |
| Cost of 1 kg pharm. preparation | Rub. | - - - | - - - | 340.00 | 200.00 | 160.00 | 200.00 | 90.00 |
| Production costs of weight gain, total | Rub. | 2159.57 | 1760.22 | 1895.21 | 1938.56 | 2116.26 | 2030.03 | 1958.36 |
| Incl. forage cost | Rub. | 1511.70 | 1232.16 | 1287.69 | 1310.35 | 1420.70 | 1341.44 | 1342.19 |
| Incl. cost of pharm. preparation | Rub. | - - - | - - - | 55.68 | 66.63 | 86.69 | 113.68 | 40.95 |
| Incl. other direct expenditure and overheads | Rub. | 647.87 | 528.07 | 551.87 | 561.58 | 608.87 | 574.90 | 575.22 |
| Prime cost per 1 kg weight gain | Rub. | 30.01 | 36.92 | 35.55 | 32.90 | 33.42 | 33.76 | 33.78 |
| Economic benefit per 1000 birds | Rub. % | +13090 | - - - | +1920 15 | +6240 48 | +5830 45 | +5000 38 | +4790 37 |

In conditions of industrial poultry husbandry, the positive effect (return) of use of studied pharmaceutical preparations as a supplement to the mycotoxin-contaminated rations can exceed 2.6 - 3.6-fold the direct costs on the purchase of these preparations, whereas the return on "Polyphepan" use will be 4-5-fold due to the lower cost of the latter.

### 2.3 CONCLUSIONS

The following conclusions can be drawn from the study conducted:
1. The inclusion into nutritionally balanced contaminated broiler chicken feed of complex preparation "Ecofiltrum" exerted a positive effect on the main production parameters (survival and body weight) of broiler chickens. Use of this preparation at maximum doses with the purpose to overcome the decline in productivity due to the presence in forages of the most common mycotoxins in high concentration (12.8 MRL), sufficiently surpassed the performance of its analogue "Polyphepan".
2. Broiler chickens suffering from chronic forms of mycotoxicosis consumed and assimilated nutrients contained in the contaminated feed supplemented with "Ecofiltrum" at doses 2.5 - 4.5 kg/t much more efficiently as compared to their counterparts consuming the analogous feedstuff lacking the pharmaceutical preparation, or supplemented with its low dose (1.5 kg/t). This could be the consequence of the more effective consumption of forage by broiler chickens over their breeding period and a higher cost return. Overall, by using different doses of complex pharmaceutical preparation it turned out to make possible to minimize the consequences of chronic mycotoxicosis, on average, by 40 - 60%. Lactulose, the prebiotic component, has proven itself effective at the backdrop of comparatively high concentrations of mycotoxins in the forages.
3. The inclusion in contaminated feed of all tested doses of lignin along with lactulose or fructooligosaccharides resulted in increased microbiological degradation of mycotoxins in gastrointestinal tract. Reduced toxic load on the avian body, along with the excretion of a fraction of toxins with the sorbent, appeared to be a favorable condition for normalization of the majority of metabolic parameters, i.e., protein biosynthesis has become more intense, the oxidation-reduction processes were optimized, broiler chickens more efficiently used and deposited fat- and water-soluble vitamins and demonstrated higher non-specific resistance which, on the whole, formed a physiological and biochemical basis for the increased production of broilers fed on the poor quality forage.
4. Use of two "Ecofiltrum" combinations to supplement the mycotoxin-contaminated compound feedstuff for broilers yielded the cost effectiveness values of over 6000 rub. as calculated per 1000 broiler chickens. It was possible to compensate by 45 - 48% the damage caused by high mycotoxin content in the forages.

Therefore, we have created an effective pharmaceutical composition for prophylaxis and treatment of gastrointestinal diseases and intoxications of diverse etiologies in animals, as well as the methods of prophylaxis and treatment using the abovementioned pharmaceutical composition.

This pharmaceutical composition secures more effective treatment and is free from the abovementioned disadvantages of hydrolyzed lignin, activated charcoal and other well known sorbents. At the same time, co-administration of lignin and prebiotic for prophylaxis and treatment of animals enables to produce a number of positive effects, namely, the minimization of sorbent side effects, restoration of gastrointestinal homeostasis and normalization of metabolism, which eventually leads to quality improvement of zootechnical and economic parameters of animal husbandry.

## Claims

1. Veterinary pharmaceutical composition for treatment of GIT diseases and intoxications of various etiologies in animals, which contains as active ingredients hydrolyzed lignin and a prebiotic of the following group: lactulose, fructooligosaccharides, galactosaccharides, inulin, made in the form suitable for use in the compound feed mixture, with lignin content from 30 to 95 % by weight, and prebiotic content from 5 to 50 % by weight.

2. The claimed composition of claim 1 **characterized in that** it is prepared in the form of powder or granules.

3. Method of prophylaxis and treatment of intoxications of various etiologies, inclusive of mycotoxicoses, in animals, including birds, during which the animals are administered the veterinary pharmaceutical composition as in claim 1, mixed with the compound feed at a dose from 2 to 4.5 kg of the preparation per 1 ton of compound feed proportionally to the content of mycotoxins in the compound feed.

4. Method of prophylaxis and treatment of gastrointestinal diseases, inclusive of dyspepsia, gastroenteritis, enteritis, colitis, hepatitis and hepatic dystrophy, inclusive of its toxic form, in animals *including* cattle and pigs, during which the animals are administered the veterinary pharmaceutical composition as in claim 1, 30 ± 10 minutes before animal feeding, orally, individually or using a group method, with water for watering or with the feedstuff, one - two times a day at a dose from 0.2 to 0.3 g/kg of animal body weight.
